(19) Europäisches Patentamt
European Patent Office
Office européen des brevets

(11) Veröffentlichungsnummer: **0 110 298**
**B1**

# (12) EUROPÄISCHE PATENTSCHRIFT

(45) Veröffentlichungstag der Patentschrift: **01.06.88**

(21) Anmeldenummer: **83111665.2**

(22) Anmeldetag: **22.11.83**

(51) Int. Cl.⁴: **C 07 D 417/12,** A 61 K 31/435 // C07D219/10, C07D219/06, (C07D417/12, 277:00, 219:10)

## (54) Acridanon-Derivate.

(30) Priorität: **26.11.82 CH 6896/82**

(43) Veröffentlichungstag der Anmeldung: **13.06.84 Patentblatt 84/24**

(45) Bekanntmachung des Hinweises auf die Patenterteilung: **01.06.88 Patentblatt 88/22**

(84) Benannte Vertragsstaaten: **AT BE CH DE FR GB IT LI LU NL SE**

(56) Entgegenhaltungen:
**DE - A - 1 811 409**
**GB - A - 1 068 595**

(73) Patentinhaber: **F. HOFFMANN-LA ROCHE & CO. Aktiengesellschaft, CH-4002 Basel (CH)**

(72) Erfinder: **Brombacher, Urs, Im Hirshalm 35, CH-4125 Riehen (CH)**
Erfinder: **Link, Helmut, Dr., Dammerkirchstrasse 70, CH-4056 Basel (CH)**
Erfinder: **Montavon, Marc, Dr., Realpstrasse 72, CH-4054 Basel (CH)**

(74) Vertreter: **Lederer, Franz, Dr. et al, Van der Werth, Lederer & Riederer Patentanwälte Lucile-Grahn-Strasse 22, D-8000 München 80 (DE)**

EP 0 110 298 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Acridanon-Derivate der allgemeinen Formel

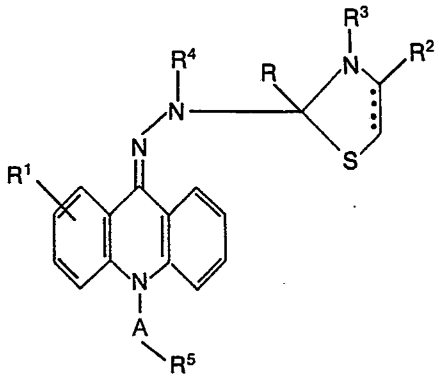

I

worin die punktierte Linie eine fakultative Bindung, $R^1$ Wasserstoff, Halogen oder Nitro, $R^2$ Wasserstoff oder niederes Alkyl, eines der Symbole $R^3$ und $R^4$ Wasserstoff oder niederes Alkyl und das andere zusammen mit R eine zusätzliche Bindung, A niederes Alkylen, $R^5$ einen 5-gliedrigen, stickstoffhaltigen, aromatischen, gegebenenfalls durch niederes Alkyl substituierten Heterocyclus, Amino oder eine Gruppe

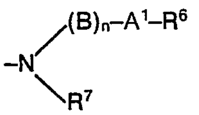 oder 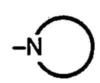 ,

das Symbol 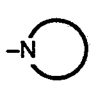

einen 5- oder 6-gliedrigen, gegebenenfalls durch niederes Alkyl substituierten gesättigten Heterocyclus, der als Ringglied noch ein Sauerstoff- oder Schwefelatom oder die Gruppe >NH oder $>N(B)_n-A^1-R^6$ enthalten kann, B die Gruppe −CO−, −COO− oder $-SO_2-$, n die Zahl 0 oder 1, $A^1$ niederes Alkylen, $R^6$ Wasserstoff, Amino, niederes Alkylamino oder Di(niederes Alkyl)amino und $R^7$ Wasserstoff oder niederes Alkyl bedeuten,
und pharmazeutisch annehmbare Säureadditionssalze davon.

Diese neuen Stoffe besitzen wertvolle pharmakologische, insbesondere schistosomizide Eigenschaften und können bei der Bekämpfung oder Verhütung von Krankheiten, insbesondere Schistosomiasen, verwendet werden.

In DE-A-1 811 409 werden 5-unsubstituierte 3-Hydrazino-Acridinderivate als Insekten- und Milbenbekämpfungsmittel beschrieben.

Gegenstand der vorliegenden Erfindung sind Acridanon-Derivate der obigen Formel I und ihre pharmazeutisch annehmbaren Salze als solche und als pharmazeutische Wirkstoffe, die Herstellung dieser Stoffe, diese enthaltende Arzneimittel und deren Verwendung zur Herstellung solcher Arzneimittel.

Die Verbindungen der obigen Formel I können, je nach Bedeutung der punktierten Linie und der Symbole R, $R^3$ und $R^4$, in verschiedenen tautomeren Formen vorliegen; die vorliegende Erfindung umfasst sämtliche möglichen tautomeren Formen.

Der Ausdruck «nieder», wie er in der vorliegenden Beschreibung verwendet wird, bedeutet, dass die derart bezeichneten Verbindungen oder Reste bis zu 7, vorzugsweise bis zu 4 Kohlenstoffatome enthalten, wobei sie geradkettig oder verzweigt sein können. Der Ausdruck «niederes Alkyl» bezeichnet gesättigte $C_1$–$C_7$-Kohlenwasserstoffreste, wie Methyl, Äthyl, Propyl, Isopropyl, n-Butyl, n-Pentyl und dergleichen. Der Ausdruck «niederes Alkylen» bezeichnet zweiwertige $C_1$–$C_7$-Kohlenwasserstoffreste, wie Methylen, Dimethylen, Trimethylen, 1,2-Propylen, 1,4-Butylen, 1,5-Butylen und dergleichen. Der Ausdruck «Halogen» bedeutet Fluor, Chlor, Brom oder Jod.

Der Ausdruck «5-gliedriger, stickstoffhaltiger, aromatischer Heterocyclus» bezeichnet aromatische Heterocyclen mit 1 bis 2 Heteroatomen, wobei, sofern 2 Heteroatome vorhanden sind, 1 Heteroatom von Stickstoff verschieden und beispielsweise ein Sauerstoff- oder Schwefelatom sein kann. Diese Heterocyclen können über ein Kohlenstoffatom oder gegebenenfalls auch über ein Stickstoffatom mit der Gruppe A verknüpft sein. Als Beispiele für in Frage kommende Heterocyclen seien die folgenden erwähnt: 1-Methyl-4-imidazolyl, 2-Pyrrolyl, 2-Thioazolyl, 4-Oxazolyl, 1-Pyrazolyl, 3-Isoxazolyl.

Der Ausdruck «5- oder 6-gliedriger gesättigter Heterocyclus, der als Ringglied noch ein Sauerstoff- oder Schwefelatom oder die Gruppe >NH oder $>N(B)_n-A^1-R^6$ enthalten kann» bezeichnet also 5- oder 6-gliedrige gesättigte Heterocyclen, welche entweder nur 1 Heteroatom, nämlich das mit A verknüpfte Stickstoffatom enthalten, wie 1-Pyrrolidinyl und 1-Piperidinyl, oder welche 2 Heteroatome enthalten, nämlich ausser dem mit A verknüpften Stickstoffatom zusätzlich 1 Sauerstoff-, Schwefel- oder Stickstoffatom, wie 4-Morpholinyl, 3-Thiazolidinyl und 1-Piperazinyl, wobei letztere am zweiten Stickstoffatom noch durch die Gruppe $-(B)_n-A^1-R^6$ substituiert sein können.

Ein allfälliger Substituent $R^1$ befindet sich vorzugsweise in der 1-Stellung. Die bevorzugte Bedeutungsmöglichkeit von $R^1$ ist jedoch Wasserstoff. $R^2$ bedeutet vorzugsweise Wasserstoff. Vorzugsweise bedeutet eines der Symbole $R^3$ und $R^4$ Wasserstoff und das andere zusammen mit R eine zusätzliche Bindung. A bedeutet vorzugsweise Dimethylen oder Trimethylen.

$R^5$ bedeutet vorzugsweise Amino, 1-Pyrrolidinyl, 1-Piperidinyl, 1-Morpholinyl, 1-Piperazinyl oder die Gruppe $-N(R^7)-A^1-R^{61}$ oder

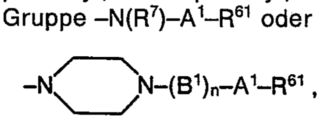 ,

wobei $A^1$ niederes Alkylen, $B^1$ die Gruppe −CO−, n die Zahl 0 oder 1, $R^{61}$ Wasserstoff und $R^7$ Wasserstoff oder niederes Alkyl bedeuten. Die besonders bevorzugten Bedeutungsmöglichkeiten von $R^5$ sind Amino, Dimethylamino, Diäthylamino, 1-Pyrrolidinyl, 1-Piperidinyl, 4-Methyl-1-piperazinyl und 4-Acetyl-1-piperazinyl.

Besonders bevorzugte, von der allgemeinen Formel I umfasste Verbindungen sind:
10-[2-(4-Methyl-1-piperazinyl)äthyl]-9-acridanon-(2-thiazolyl)hydrazon,
10-[2-(4-Methyl-1-piperazinyl)äthyl]-9-acridanon-(2-thiazolidinyliden)hydrazon,
10-[2-(4-Acetyl-1-piperazinyl)äthyl]-9-acridanon-(2-thiazolidinyliden)hydrazon,
10-[2-(Dimethylamino)äthyl]-9-acridanon-(2-thiazolidinyliden)hydrazon,
10-[2-(Diäthylamino)äthyl]-9-acridanon-(2-thiazolyl)hydrazon,
10-[2-(1-Piperidinyl)äthyl]-9-acridanon-(2-thiazolyl)hydrazon,
10-(2-Aminoäthyl)-9-acridanon-(2-thiazolyl)hydrazon,
10-[2-(Dimethylamino)äthyl]-9-acridanon-(2-thiazolyl)hydrazon,
10-[2-(4-Acetyl-1-piperazinyl)äthyl]-9-acridanon-(2-thiazolyl)hydrazon,
10-[3-(Dimethylamino)propyl]-9-acridanon-(2-thiazolyl)hydrazon,
10-[2-(Diäthylamino)äthyl]-9-acridanon-(2-thiazolidinyliden)hydrazon,
10-[2-(1-Pyrrolidinyl)äthyl]-9-acridanon-(2-thiazolidinyliden)hydrazon und
10-[3-(Dimethylamino)propyl]-9-acridanon-(2-thiazolidinyliden)hydrazon.

Weitere repräsentative Vertreter der durch die allgemeine Formel I definierten Stoffklasse sind:
10-[2-(4-Propyl-1-piperazinyl)äthyl]-9-acridanon-(2-thiazolidinyliden)hydrazon,
10-[2-(Dimethylamino)propyl]-9-acridanon-(2-thiazolidinyliden)hydrazon,
10-[2-(1-Piperidinyl)äthyl]-9-acridanon-(2-thiazolidinyliden)hydrazon,
10-[2-(2,6-Dimethyl-1-piperidinyl)-äthyl]-9-acridanon-(2-thiazolidinyliden)hydrazon,
10-[2-(4-Morpholinyl)äthyl]-9-acridanon-(2-thiazolidinyliden)hydrazon,
10-[2-(4-Äthoxycarbonyl-1-piperazinyl)-äthyl]-9-acridanon-(2-thiazolidinyliden)-hydrazon,
10-[2-(4-Pivaloyl-1-piperazinyl)äthyl]-9-acridanon-(2-thiazolidinyliden)hydrazon,
10-[2-[4-(Methylsulfonyl)-1-piperazinyl]-äthyl]-9-acridanon-(2-thiazolidinyliden)-hydrazon,
10-[(1-Methyl-4-imidazolyl)methyl]-9-acridanon-(2-thiazolidinyliden)hydrazon,
10-[2-[4-[2-(Dimethylamino)acetyl]-1-piperazinyl]-äthyl]-9-acridanon-(2-thiazolidinyliden)hydrazon,
1-Chlor-10-[2-(diäthylamino)äthyl]-9-acridanon-(2-thiazolidinyliden)hydrazon,
10-(2-Aminoäthyl)-9-acridanon-(2-thiazolidinyliden)hydrazon,
10-[2-[(Methylsulphonyl)amino]äthyl]-9-acridanon-(2-thiazolidinyliden)hydrazon,
10-[4-(Diäthylamino)butyl]-9-acridanon-(2-thiazolidinyliden)hydrazon,
10-[5-(Diäthylamino)pentyl]-9-acridanon-(2-thiazolidinyliden)hydrazon,
10-[2-(Diäthylamino)äthyl]-9-acridanon-methyl-(2-thiazolin-2-yl)hydrazon,
10-[2-(Diäthylamino)äthyl]-9-acridanon-(3-methyl-2-thiazolidinyliden)hydrazon,
10-[2-(Diäthylamino)äthyl]-9-acridanon-methyl(2-thiazolyl)hydrazon,
10-[2-(4-Morpholinyl)äthyl]-9-acridanon-(2-thiazolyl)hydrazon,
10-[2-(4-Äthoxycarbonyl-1-piperazinyl]-äthyl-9-acridanon-(2-thiazolyl)hydrazon,
10-[2-(4-Pivaloyl-1-piperazinyl)äthyl]-9-acridanon-(2-thiazolyl)hydrazon,
10-[2-[4-(Methylsulphonyl)-1-piperazinyl]-äthyl]-9-acridanon-(2-thiazolyl)hydrazon,
10-[2-[4-[2-(Diäthylamino)äthyl]-1-piperazinyl]äthyl]-9-acridanon-(2-thiazolyl)-hydrazon und
10-[2-(4-Propyl-1-piperazinyl)äthyl]-9-acridanon-(2-thiazolyl)hydrazon.

Die Acridanon-Derivate der eingangs definierten Formel I und ihre pharmazeutisch annehmbaren Säureadditionssalze können erfindungsgemäss dadurch hergestellt werden, dass man

a) eine Verbindung der allgemeinen Formel

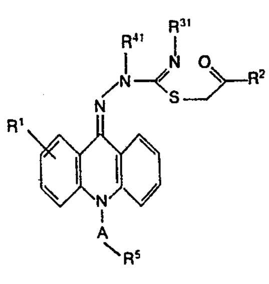
II,
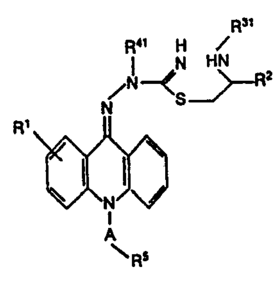
III,
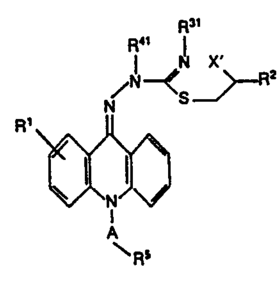
IV,
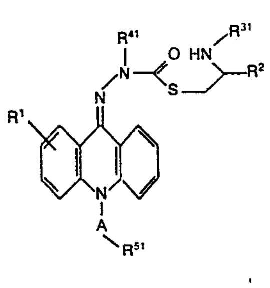
V,
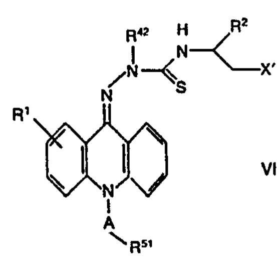
VI oder
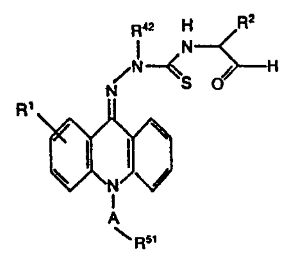
VII

worin eines der Symbole $R^{31}$ und $R^{41}$ Wasserstoff oder niederes Alkyl und das andere Wasserstoff, $R^{42}$ Wasserstoff oder niederes Alkyl, $R^{51}$ einen wie oben definierten Rest $R^5$, der jedoch keine primäre oder sekundäre, basische Aminogruppe enthält, und X′ eine Abgangsgruppe bedeuten, und R, $R^1$, $R^2$ und $R^5$ obige Bedeutung besitzen, cyclisiert, oder

b) eine Verbindung der allgemeinen Formel

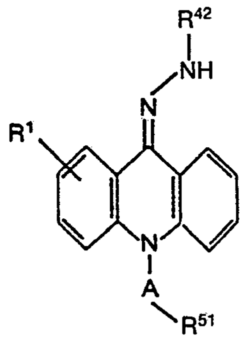

VIII

worin A, $R^1$, $R^{42}$ und $R^{51}$ obige Bedeutung besitzen, mit einer Verbindung der allgemeinen Formel

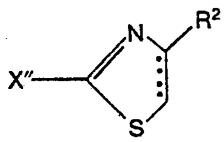

IX

worin X″ eine Abgangsgruppe bedeutet, und die punktierte Linie und $R^2$ obige Bedeutung besitzen, umsetzt, oder

c) eine Verbindung der allgemeinen Formel

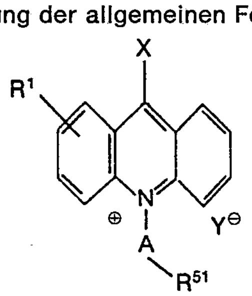

X

worin $Y^{\ominus}$ ein Anion und X eine Abgangsgruppe bedeuten, und A, $R^1$ und $R^{51}$ obige Bedeutung besitzen, mit einer Verbindung der allgemeinen Formel

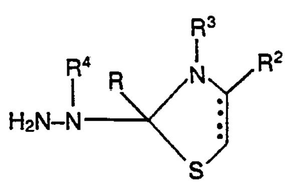

XI

worin die punktierte Linie, R, $R^2$, $R^3$ und $R^4$ obige Bedeutung besitzen,
umsetzt, oder

d) die N-Schutzgruppe in einer Verbindung der allgemeinen Formel

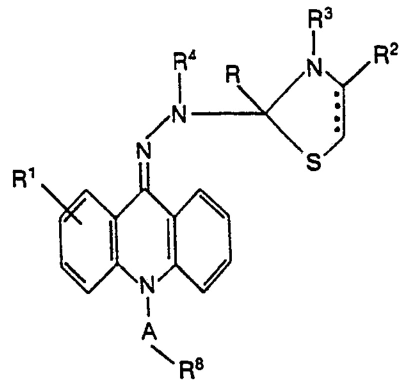

XII

worin $R^8$ einen wie oben definierten Rest $R^5$ bedeutet, welcher eine durch eine N-Schutzgruppe blockierte primäre oder sekundäre, basische Aminogruppe enthält, und die punktierte Linie, R, $R^1$, $R^2$, $R^3$ und $R^4$ obige Bedeutung besitzen, abspaltet, und

e) erwünschtenfalls eine erhaltene Verbindung der Formel I in ein pharmazeutisch annehmbares Säureadditionssalz überführt.

Gemäss Verfahrensvariante a) können die Verbindungen der Formel I hergestellt werden, indem man eine Verbindung der Formel II, III, IV, V, VI oder VII nach an sich bekannten und jedem Fachmann geläufigen Methoden cyclisiert. Die in den Formeln IV und VI mit X′ bezeichnete Abgangsgruppe ist vorzugsweise ein Halogenatom, insbesondere ein Brom- oder Chloratom. Die Ringschlussreaktion erfolgt je nach verwendetem Ausgangsstoff ziemlich leicht und kann gegebenenfalls durch längeres Stehenlassen und/oder Anwendung von Wärme bewerkstelligt oder vervollständigt werden. Die für die Ringschlussreaktion verwendeten Ausgangsstoffe müssen nicht notwendigerweise in isoliertem Zustand eingesetzt werden; in der Regel erweist es sich als zweckmässig, diese Ausgangsstoffe ohne Isolierung aus dem Reaktionsgemisch, in dem sie hergestellt worden sind, direkt zu cyclisieren bzw. cyclisieren zu lassen. Je nach angewandten Reaktionsbedingungen ist in einigen Fällen eine Isolierung gar nicht möglich, da die Cyclisierung spontan erfolgt.

Für den vorliegenden Verfahrensaspekt geeignete Lösungsmittel sind beispielsweise Äther, wie Tetrahydrofuran, Dioxan, Äthylenglykol-dimethyläther, Diäthylenglykol-dimethyläther und dergleichen, Alkohole, wie Methanol, Äthanol und dergleichen, Dimethylformamid, Dimethylsulfoxid, Acetonitril und dergleichen. Die Reaktionstemperatur liegt zweckmässigerweise in einem Bereich von etwa Raumtemperatur bis zur Siedetemperatur der Reaktionsmischung.

Gemäss Verfahrensvariante b) können die Verbindungen der Formel I durch Umsetzen einer Verbindung der Formel VIII mit einer Verbindung der Formel IX erhalten werden. Die in der Formel IX mit X″ bezeichnete Abgangsgruppe ist vorzugsweise ein Halogenatom, z.B. ein Chlor- oder Bromatom, oder die Thiolgruppe. Die folgenden, unter den Reaktionsbedingungen inerten organischen Lösungsmittel können verwendet werden: Äther, wie Tetrahydrofuran, Dioxan, Diäthyläther und dergleichen, Alkohole, wie Methanol, Äthanol und dergleichen, Dimethylformamid, Dimethylsulfoxid, Acetonitril und dergleichen. Die Reaktionstemperatur liegt zweckmässigerweise in einem Bereich von etwa Raumtemperatur bis zur Siedetemperatur der Reaktionsmischung.

Gemäss Verfahrensvariante c) können die Verbindungen der Formel I dadurch hergestellt werden, dass man eine Verbindung der Formel X mit einer Verbindung der Formel XI umsetzt. Die in der Verbindung der Formel X mit X bezeichnete Abgangsgruppe ist vorzugsweise ein Halogenatom, eine niedere Alkanoyloxygruppe oder eine niedere Alkoxygruppe, insbesondere ein Chloratom, eine Acetyloxygruppe oder eine Methoxygruppe. Bei den Verbindungen der Formel X handelt es sich zum Teil um Substanzen, welche nicht besonders stabil sind. Sie werden deshalb zweckmässigerweise kurz vor der Reaktion mit einer Verbindung der Formel XI aus einer Verbindung der allgemeinen Formel

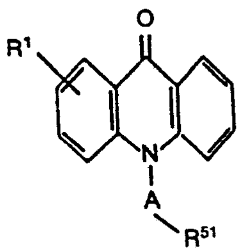

XIV

worin A, $R^1$ und $R^{51}$ obige Bedeutung besitzen, wie weiter unten beschrieben hergestellt und, gegebenenfalls ohne vorgängige Isolierung, direkt weiterverarbeitet.

Die Verbindung der Formel XI wird zweckmässigerweise in Form eines Säureadditionssalzes eingesetzt, beispielsweise in Form eines Hydrochlorides oder Hydrobromides. Man kann die Reaktion in Gegenwart eines säurebindenden Mittels durchführen, wobei als säurebindende Mittel insbesondere Natrium- und Kaliumcarbonate, -bicarbonate und -acetate in Frage kommen. Als Lösungsmittel für den vorliegenden Verfahrensaspekt eignen sich beispielsweise niedere Alkohole, wie Methanol und Äthanol, und andere unter den Reaktionsbedingungen inerte organische Lösungsmittel, wie Dimethylformamid, Acetonitril und dergleichen. Die Reaktionstemperatur liegt zweckmässigerweise in einem Bereich von etwa Raumtemperatur bis zur Siedetemperatur der Reaktionsmischung.

Gemäss Verfahrensvariante d) können die Verbindungen der Formel I, welche im Rest $R^5$ eine primäre oder sekundäre, basische Aminogruppe besitzen, hergestellt werden, indem man in einer Verbindung der Formel XII die N-Schutzgruppe abspaltet. Als Schutzgruppen eignen sich für die Zwecke der vorliegenden Erfindung in erster Linie Acylgruppen, vorzugsweise leicht abspaltbare Alkoxycarbonyl- oder gegebenenfalls am Phenylring substituierte Phenylalkoxycarbonylgruppen, insbesondere die t-Butoxycarbonylgruppe, die Benzyloxycarbonylgruppe usw., ferner auch leicht abspaltbare, am Phenylring gegebenenfalls substituierte Phenylalkylgruppen, wie die Benzylgruppe. Die Abspaltung der Schutzgruppe erfolgt nach an sich bekannten Methoden, wobei natürlich die Natur der zu entfernenden Schutzgruppe für die Wahl der zur Anwendung gelangenden Methode in Betracht gezogen werden muss. Ebenfalls zu beachten ist natürlich, dass nur solche Methoden verwendet werden können, welche die Schutzgruppe selektiv entfernen, ohne dass andere im Molekül vorhandene Strukturelemente in Mitleidenschaft gezogen werden.

Die weiter oben als Beispiele für in Betracht kommende Schutzgruppen erwähnten Reste können hydrolytisch abgespalten werden. So können z.B. die Benzyloxycarbonylgruppe und die t-Butoxycarbonylgruppe unter selektiven sauren Bedingungen abgespalten werden, z.B. durch Behandeln mit einem Gemisch von Bromwasserstoff und Eisessig oder durch Behandeln mit Bortrifluorid oder Bortribromid in einem inerten organischen Lösungsmittel, wie Dichlormethan. Die t-Butoxycarbonylgruppe kann auch durch Behandeln mit Chlorwasserstoff in einem inerten organischen Lösungsmittel, wie Dioxan, Tetrahydrofuran oder dergleichen, oder durch Behandeln mit Trifluoressigsäure abgespalten werden.

Gemäss Verfahrensvariante e) können die Acridanon-Derivate der eingangs definierten Formel I in pharmazeutisch annehmbare Säureadditionssalze übergeführt werden. Die Herstellung von solchen Säureadditionssalzen erfolgt dabei nach allgemein üblichen Methoden. Es kommen sowohl Salze mit pharmazeutisch annehmbaren anorganischen als auch Salze mit pharmazeutisch annehmbaren organischen Säuren in Betracht, beispielsweise Hydrochloride, Hydrobromide, Sulfate, Citrate, Acetate, Succinate, Methansulfonate, p-Toluolsulfonate und dergleichen.

Die als Ausgangsstoffe verwendeten Verbindungen der Formel X, sowie diejenigen der Formeln II, III, IV, V, VI, VII, VIII und XII können ausgehend von Verbindungen der Formel XIII gemäss dem nachfolgenden Formelschema hergestellt werden; die punktierte Linie und die Symbole A, $R^1$, $R^2$, $R^{31}$, $R^{41}$, $R^{42}$, $R^{51}$, $R^8$, X und X′ besitzen obige Bedeutung, $R^9$ und $R^{91}$ bedeuten je niederes Alkyl oder zusammen niederes Alkylen, $R^{52}$ bedeutet einen Rest $R^5$, welcher eine primäre oder sekundäre basische Aminogruppe enthält, und $R^{10}$ bedeutet niederes Alkyl, Phenyl oder substituiertes Phenyl. Die Verbindungen der Formel XIII gehören einer an sich bekannten Stoffklasse an.

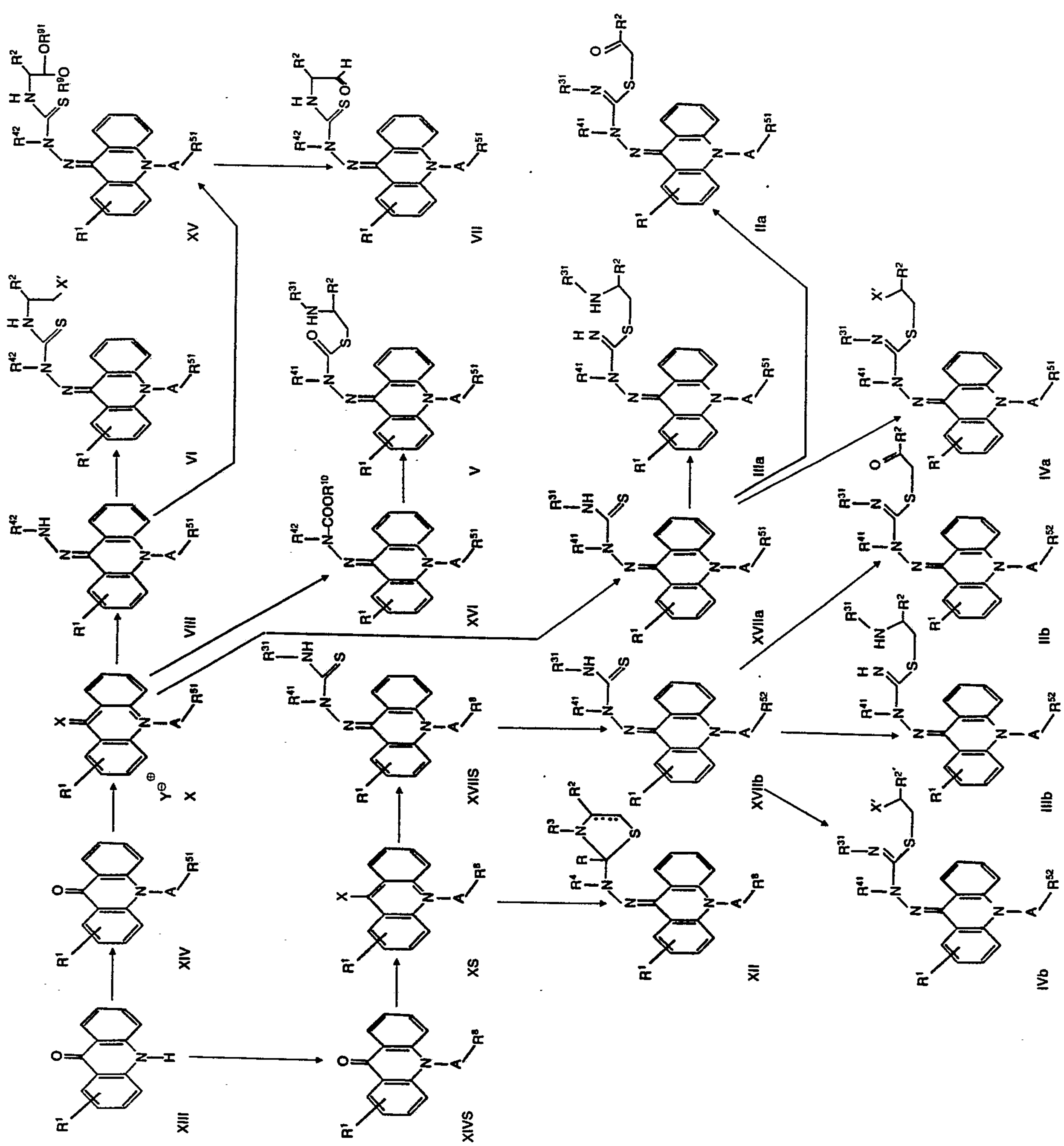

XV VII IIa VI V IIIa IVa VIII XVI XVIIa IIb X XVIIIS XVIIb IIIb XIV XS XII IVb XIII XIVS

Die Verbindungen der Formel XIV können aus Verbindungen der Formel XIII hergestellt werden, indem man eine Verbindung der Formel XIII in Gegenwart einer starken Base, wie Natriumhydrid oder dergleichen, mit einem den Rest $-A-R^{51}$ liefernden Mittel alkyliert. Diese Reaktion erfolgt nach an sich bekannten und jedem Fachmann geläufigen Methoden.

Diejenigen Verbindungen der Formel X, worin X ein Halogenatom bedeutet, können hergestellt werden, indem man eine Verbindung der Formel XIV in einem inerten organischen Lösungsmittel mit einem Halogenierungsmittel behandelt. In einer bevorzugten Ausführungsform verwendet man als Halogenierungsmittel Oxalylchlorid oder Phosphoroxychlorid und als Lösungsmittel halogenierte Kohlenwasserstoffe, wie Methylenchlorid, Chloroform, 1,2-Dichloräthan oder dergleichen, Acetonitril oder überschüssiges Halogenierungsmittel, wobei man eine Verbindung der Formel X erhält, worin X Chlor bedeutet. Die Reaktionstemperatur variiert vorteilhafterweise in einem Bereich von etwa Raumtemperatur bis zur Siedetemperatur der Reaktionsmischung.

Verbindungen der Formel X, worin X eine von Halogen verschiedene Abgangsgruppe bedeutet, können aus den entsprechenden Halogenverbindungen erhalten werden. Man kann beispielsweise das Halogenatom in einer solchen Verbindung in an sich bekannter Weise durch andere Abgangsgruppen, z. B. durch niedere Alkoxygruppen oder niedere Alkanoyloxygruppen, ersetzen.

Bei den Verbindungen der Formel X handelt es sich um quaternäre Ammoniumsalze, wovon einige, wie bereits früher erwähnt, nicht besonders stabil sind; diese werden zweckmässigerweise unmittelbar nach ihrer Herstellung weiterverarbeitet. Die Natur des mit $Y^{\ominus}$ bezeichneten Anions hängt von der Art der Herstellung der entsprechenden Verbindung der Formel X ab. Stellt man beispielsweise eine Verbindung der Formel X her, worin X Chlor bedeutet, und verwendet als Halogenierungsmittel Oxalylchlorid, so erhält man eine Verbindung der Formel X, worin $Y^{\ominus}$ ein Chloridanion ist; verwendet man als Halogenierungsmittel Phosphoroxychlorid so erhält man eine entsprechende Verbindung, worin $Y^{\ominus}$ $PO_2Cl_2^{\ominus}$ bedeutet.

Diejenigen Verbindungen der Formel II, worin der Rest $R^5$ keine primäre oder sekundäre, basische Aminogruppe enthält, d.h. Verbindungen der Formel IIa, können hergestellt werden, indem man eine Verbindung der Formel X mit einem Thiosemicarbazid der allgemeinen Formel

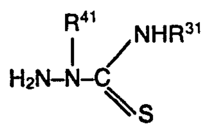

XVIII

worin $R^{31}$ und $R^{41}$ obige Bedeutung besitzen, umsetzt, wobei man die weiter oben für die Verfahrensvariante c) angegebenen Reaktionsbedingungen anwenden kann, und die erhaltene Verbindung der allgemeinen Formel XVIIa mit einer Verbindung der allgemeinen Formel

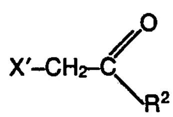

XIX

worin X′ und $R^2$ obige Bedeutung besitzen, umsetzt. Die mit X′ bezeichnete Abgangsgruppe ist vorzugsweise ein Chlor- oder Bromatom. Als Lösungsmittel eignen sich insbesondere niedere Alkohole, wie Methanol und Äthanol. Die Reaktionstemperatur liegt zweckmässigerweise in einem Bereich von etwa Raumtemperatur bis Siedetemperatur der Reaktionsmischung.

Die Verbindungen der Formel II, worin $R^5$ eine primäre oder sekundäre, basische Aminogruppe enthält, können hergestellt werden, indem man eine Verbindung der Formel XIII (in Analogie zur Herstellung einer Verbindung der Formel XIV) mit einem den Rest $-A-R^8$ liefernden Mittel alkyliert, die erhaltene Verbindung der Formel XIVS (in Analogie zur Herstellung der Verbindungen der Formel X) in eine Verbindung der Formel XS überführt, diese (in Analogie zur Herstellung der Verbindungen der Formel XVIIa) mit einem Thiosemicarbazid der Formel XVIII umsetzt, in der erhaltenen Verbindung der allgemeinen Formel XVIIS (unter Anwendung der weiter oben für die Verfahrensvariante d) angegebenen Reaktionsbedingungen) die N-Schutzgruppe abspaltet und die erhaltene Verbindung der Formel XVIIb (in Analogie zur Herstellung der Verbindungen der Formel IIa) mit einer Verbindung der Formel XIX umsetzt.

Die Verbindungen der Formel III können erhalten werden, indem man eine Verbindung der Formel XVIIa oder XVIIb, worin $R^{31}$ jeweils Wasserstoff bedeutet, mit einer Verbindung der allgemeinen Formel

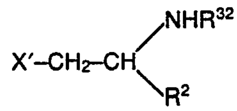

XX

worin X′ und $R^2$ obige Bedeutung besitzen, und $R^{32}$ Wasserstoff oder, falls $R^{41}$ in der Verbindung der Formel XVIIa oder XVIIb Wasserstoff bedeutet, auch niederes Alkyl bedeutet,
umsetzt. Das Amin wird dabei zweckmässigerweise in Form eines Säureadditionssalzes eingesetzt, wobei insbesondere die Hydrochloride oder Hydrobromide in Frage kommen. Als Lösungsmittel eignen sich beispielsweise niedere Alkohole, wie Methanol und Äthanol, Äther, wie Tetrahydrofuran, Dioxan und Äthylenglykol-dimethyläther, Dimethylformamid, Dimethylsulfoxid, Acetonitril und dergleichen. Die Reaktionstemperatur liegt vorzugsweise zwischen etwa Raumtemperatur und Siedetemperatur der Reaktionsmischung.

Die Verbindungen der Formel IV können erhalten werden, indem man eine Verbindung der Formel XVIIa oder XVIIb in einem inerten organischen Lösungsmittel und in einem Bereich von etwa Raumtemperatur bis Siedetemperatur der Reaktionsmischung mit einer Verbindung der allgemeinen Formel

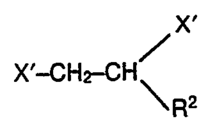

XXI

worin X′ und $R^2$ obige Bedeutung besitzen,
umsetzt, wobei man vorzugsweise ein Dichlorid oder Dibromid verwendet. Geeignete Lösungsmittel sind beispielsweise Alkohole, wie Methanol, Äthanol und dergleichen. Äther, wie Tetrahydrofuran, Dioxan und dergleichen, Dimethylformamid, Dimethylsulfoxid und dergleichen.

Die Verbindungen der Formel V können hergestellt werden, indem man eine Verbindung der Formel X mit einem Hydrazin der allgemeinen Formel

$$H_2N-NHR^{42} \qquad XXII$$

worin $R^{42}$ obige Bedeutung besitzt,
umsetzt, wobei man die weiter oben für die Verfahrensvariante c) angegebenen Reaktionsbedingungen anwenden kann, die erhaltene Verbindung der allgemeinen Formel VIII in an sich bekannter Weise mit einem den Rest $-COOR^{10}$ liefernden Mittel, z.B. einem Dialkyl- oder Diphenylcarbamat oder einem Chlorameisensäure-alkyl- oder -phenylester, behandelt und die erhaltene Verbindung der allgemeinen Formel XVI mit einem Thiol der allgemeinen Formel

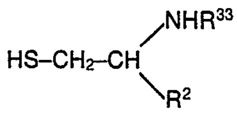

XXIII

worin $R^2$ obige Bedeutung besitzt, und $R^{33}$ Wasserstoff oder, falls $R^{42}$ in der Verbindung der Formel XVI Wasserstoff bedeutet, auch niederes Alkyl bedeutet,

umsetzt. Dieser dritte Schritt wird vorzugsweise zwischen etwa Raumtemperatur und Siedetemperatur der Reaktionsmischung in einem inerten organischen Lösungsmittel durchgeführt, wobei als Lösungsmittel insbesondere Äther, wie Diäthyläther, Tetrahydrofuran und dergleichen, und niedere Alkohole, wie Methanol und Äthanol, in Betracht kommen.

Die Verbindungen der Formel XVI können andererseits auch durch Umsetzen einer Verbindung der Formel X mit einem Hydrazin der allgemeinen Formel

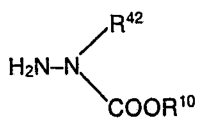

XXIV

worin $R^{42}$ und $R^{10}$ obige Bedeutung besitzen, hergestellt werden, was in an sich bekannter Weise erfolgen kann, beispielsweise unter Anwendung der weiter oben für die Verfahrensvariante c) angegebenen Reaktionsbedingungen.

Die Verbindungen der Formel VI können hergestellt werden, indem man eine Verbindung der Formel VIII mit einem Isothiocyanat der allgemeinen Formel

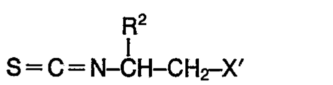

XXV

worin X′ und $R^2$ obige Bedeutung besitzen, umsetzt. Zweckmässigerweise arbeitet man bei Temperaturen von etwa Raumtemperatur bis Siedetemperatur und verwendet ein inertes organisches Lösungsmittel, z.B. einen Äther, wie Diäthyläther, t-Butylmethyläther und Tetrahydrofuran, oder Dimethylformamid, Acetonitril oder dergleichen.

Die Verbindungen der Formel VII können hergestellt werden, indem man eine Verbindung der Formel VIII mit einem Isothiocyanat der allgemeinen Formel

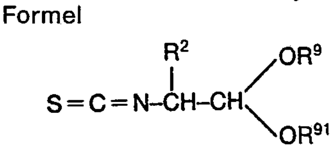

XXVI

worin $R^2$, $R^9$ und $R^{91}$ obige Bedeutung besitzen, umsetzt und anschliessend die Acetalgruppe in der erhaltenen Verbindung der allgemeinen Formel XV hydrolysiert. Der erste Schritt wird zweckmässigerweise in einem inerten organischen Lösungsmittel, z.B. in einem Äther, wie Diäthyläther, t-Butylmethyläther und Tetrahydrofuran, oder in Dimethylformamid, Acetonitril oder dergleichen, bei Temperaturen von etwa Raumtemperatur bis Siedetemperatur durchgeführt. Die Hydrolyse der Acetalgruppe kann mittels einer wässrigen Säure bewerkstelligt werden, wobei man gegebenenfalls in Gegenwart eines Lösungsvermittlers, wie Tetrahydrofuran, Dioxan, Methanol, Äthanol, Dimethylformamid oder dergleichen, arbeiten kann. Als Säuren kommen z.B. Schwefelsäure, Salzsäure, p-Toluolsulfonsäure und dergleichen in Betracht. Die Temperatur ist nicht kritisch und kann in einem weiten Bereich variieren.

Wie bereits weiter oben erwähnt, ist es nicht notwendig (und in manchen Fällen auch nicht möglich), die Verbindungen der Formeln II, III, IV, V, VI und VII zu isolieren; vielmehr erweist es sich in der Regel als zweckmässig, diese Verbindungen ohne Isolierung aus dem Reaktionsgemisch, in dem sie hergestellt worden sind, direkt zu cyclisieren bzw. cyclisieren zu lassen.

Die als Ausgangsstoffe verwendeten Verbindungen der Formel XII können aus Verbindungen der Formel XS hergestellt werden, und zwar in Analogie zur Herstellung der Verbindungen der Formel I aus Verbindungen der Formel X gemäss den weiter oben beschriebenen Verfahrensvarianten a), b) und c) und den für die Herstellung der entsprechenden Ausgangsstoffe beschriebenen Methoden.

Die Ausgangsstoffe der allgemeinen Formeln II, III, IV, V, VI, VII und XII sind neu.

Die Acridanon-Derivate der obigen Formel I und ihre pharmazeutisch annehmbaren Säureadditionssalze besitzen wertvolle pharmakologische Eigenschaften; sie entfalten insbesondere wertvolle schistosomizide Wirkungen und können demnach bei der Bekämpfung bzw. Verhütung von Schistosomiasen verwendet werden.

Die schistosomizide Wirkung der erfindungsgemässen Stoffe kann tierexperimentell wie folgt nachgewiesen werden:

Albino-Mäuse mit einem Gewicht von 15–18 g werden subcutan mit $60 \pm 5$ Cercarien eines Liberia-Stammes von Schistosoma mansoni infiziert. 46 Tage nach der Infektion werden die Tiere einmal mit den zu prüfenden Substanzen peroral behandelt. Pro Substanz und Dosierung werden 5–10 Tiere eingesetzt. Als Kontrolle dienen 10 unbehandelte Tiere. Die Sektion erfolgt nach 19 Tagen, worauf Wurmpaare und einzelne Würmer in Mesenterialvenen, Pfortader und Leber herauspräpariert und gezählt werden. Die vermicide Wirkung zeigt sich in einer reduzierten Zahl lebender Parasiten im Vergleich zu der Zahl in den Kontrolltieren.

Zur Auswertung wird die prozentuale Reduktion der Parasiten bei behandelten Tieren im Vergleich zu unbehandelten Kontrolltieren berechnet. Man bestimmt die $VD_{50}$ nach der Probitmethode. Die $VD_{50}$ ist diejenige vermizide Dosis, welche eine 50-proz. Reduktion der Wurmzahl bewirkt.

In der nachfolgenden Tabelle werden die Resultate dargestellt, welche mit einigen repräsentativen erfindungsgemässen Substanzen erhalten worden sind. Angegeben werden für jede der darin figurierenden Verbindung die $VD_{50}$ in mg/kg p.o. und, für einige dieser Verbindungen, die $DL_{50}$ in mg/kg bei einmaliger oraler Verabreichung an Mäusen.

Tabelle

| Verbindung der Formel I | $VD_{50}$ in mg/kg p.o. | $DL_{50}$ in mg/kg p.o. |
|---|---|---|
| 10-[2-(Diäthylamino)äthyl]-9-acridanon-(2-thiazolidinyliden)hydrazon | 9,5 | 312–625 |
| 10-[3-(Dimethylamino)propyl]-9-acridanon-(2-thiazolidinyliden)hydrazon | 4,7 | 125–250 |
| 10-[2-(4-Methyl-1-piperazinyl)äthyl]-9-acridanon-(2-thiazolidinyliden)hydrazon | 4,0 | 156–312 |
| 10-[2-(Dimethylamino)äthyl]-9-acridanon-(2-thiazolidinyliden)hydrazon | 6,4 | 250–500 |
| 10-[2-(1-Pyrrolidinyl)äthyl]-9-acridanon-(2-thiazolidinyliden)hydrazon | 6,2 | 156–312 |
| 10-[2-(4-Acetyl-1-piperazinyl)äthyl]-9-acridanon-(2-thiazolidinyliden)hydrazon | 3,4 | 1250–2500 |
| 10-[2-(Diäthylamino)äthyl]-9-acridanon-(2-thiazolyl)hydrazon/2 HCl | 3,3 | >5000 |
| 10-[3-(Dimethylamino)propyl]-9-acridanon-(2-thiazolyl)hydrazon/2 HCl | 3,5 | 312–625 |
| 10-[2-(1-Piperidinyl)äthyl]-9-acridanon-(2-thiazolyl)hydrazon/2:3 HCl | 9,0 | 312–625 |
| 10-[2-(4-Acetyl-1-piperazinyl)äthyl]-9-(acridanon-(2-thiazolyl)hydrazon/2 HCl | 2,6 | 312–625 |
| 10-(2-Aminoäthyl)-9-acridanon-(2-thiazolyl)hydrazon/2 HCl | 2,6 | 156–312 |
| 10-[2-(Dimethylamino)äthyl]-9-acridanon-(2-thiazolyl)hydrazon/2 HCl | 4,9 | 312–625 |
| 10-[2-(4-Methyl-1-piperazinyl)äthyl]-9-acridanon-(2-thiazolyl)hydrazon/2:5 HCl | 2,4 | 625–1250 |

Die erfindungsgemässen Produkte können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Öle, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Drukkes, Puffer, Überzugsmittel oder Antioxidantien enthalten. Sie können auch noch andere therapeutisch wertvolle Stoffe enthalten.

Wie eingangs erwähnt sind Arzneimittel, enthaltend ein Acridanon-Derivat der Formel I oder ein pharmazeutisch annehmbares Säureadditionssalz einer solchen Verbindung ebenfalls Gegenstand der vorliegenden Erfindung, weiterhin auch ein Verfahren zur Herstellung solcher Arzneimittel, welches dadurch gekennzeichnet ist, dass man eine oder mehrere erfindungsgemässe Stoffe und gegebenenfalls einen oder mehrere andere therapeutisch wertvolle Stoffe in eine galenische Darreichungsform bringt.

Wie eingangs erwähnt, können die erfindungsgemässen Produkte bei der Bekämpfung bzw. Verhütung von Krankheiten verwendet werden. Sie eignen sich insbesondere zur Bekämpfung bzw. Verhütung von Schistosomiasen. Die Dosierung kann innerhalb weiter Grenzen variieren und ist natürlich in jedem einzelnen Fall den individuellen Gegebenheiten anzupassen. Im allgemeinen dürfte eine einmalige Dosis von etwa 1 bis etwa 50 mg/kg Körpergewicht für die Behandlung von Schistosomiasen angemessen sein, wobei diese Dosis auch mehrfach unterteilt an einem Tag verabreicht werden kann.

Die nachfolgenden Beispiele sollen die vorliegende Erfindung näher erläutern, ihren Umfang jedoch in keiner Weise beschränken. Alle Temperaturen sind in Celsiusgraden angegeben.

Beispiel 1

a) Man rührt eine Mischung aus 8,5 g 9-Acridanon, 180 ml Dimethylformamid und 3,3 g Natriumhydrid während 0,5 Stunden, versetzt portionenweise mit 10,3 g
2-[1-(4-Methyl)-piperazinyl]äthylchlorid-dihydrochlorid,
rührt während 3 Tagen bei 80°, dampft ein, versetzt den Rückstand mit Wasser und extrahiert mit Methylenchlorid. Der Auszug wird mit Wasser gewaschen, über Natriumsulfat getrocknet und eingedampft, worauf man den Rückstand zuerst aus Äther und dann aus Essigester umkristallisiert. Man erhält
10-[2-(4-Methyl-1-piperazinyl)äthyl]-9-acridanon
vom Schmelzpunkt 156–157°.

Man versetzt eine Lösung von 2,2 g
10-[2-(4-Methyl-1-piperazinyl)äthyl]-9-acridanon
in 100 ml Dichlormethan bei –5° portionenweise mit 1,17 ml Oxalylchlorid, rührt noch während 0,5 Stunden bei Raumtemperatur und dampft ein. Man versetzt den Rückstand

(9-Chlor-10-[2-(4-methyl-1-piperazinyl)äthyl]-acridinium-chlorid)

mit 100 ml Methanol, 1,346 g Hydrazino-2-thiazolin-hydrobromid und 1,67 g Natriumacetat, erhitzt unter Rückfluss zum Sieden, kühlt nach 10 Minuten ab und dampft ein. Man versetzt den Rückstand mit gesättigter Sodalösung, extrahiert mit Methylenchlorid, wäscht den Auszug mit Wasser, trocknet diesen über Natriumsulfat und konzentriert. Nach Zugabe von Essigester wird das auskristallisierte Produkt abfiltriert und nacheinander mit Essigester und Petroläther gewaschen. Man erhält

10-[2-(4-Methyl-1-piperazinyl)äthyl]-9-acridanon-(2-thiazolidinyliden)hydrazon

vom Schmelzpunkt 204–205°.

In analoger Weise erhält man:

b) Aus 9-Acridanon und 3-(Dimethylamino)propylchlorid das 10-[3-(Dimethylamino)propyl]-9-acridanon vom Schmelzpunkt 89° und daraus das

10-[3-(Dimethylamino)propyl]-9-acridanon-(2-thiazolidinyliden)hydrazon

vom Schmelzpunkt 191–192°;

c) aus 9-Acridanon und

2-(Diäthylamino)äthylchlorid-hydrochlorid

das 10-[2-(Diäthylamino)äthyl]-9-acridanon vom Schmelzpunkt 109–111° und daraus das

10-[2-(Diäthylamino)äthyl]-9-acridanon-(2-thiazolidinyliden)hydrazon

vom Schmelzpunkt 153–155°;

d) aus 9-Acridanon und 2-(1-Pyrrolidinyl)äthylchlorid-hydrochlorid das 10-[2-(1-Pyrrolidinyl)-äthyl]-9-acridanon vom Schmelzpunkt 142–145° und daraus das

10-[2-(1-Pyrrolidinyl)äthyl]-9-acridanon-(2-thiazolidinyliden)hydrazon

vom Schmelzpunkt 220° (Zersetzung);

e) aus 9-Acridanon und

2-(1-Piperidinyl)äthylchlorid-hydrochlorid das 10-[2-(1-Piperidinyl)äthyl]-9-acridanon vom Schmelzpunkt 165° und daraus das

10-[2-(1-Piperidinyl)äthyl]-9-acridanon-(2-thiazolidinyliden)hydrazon

vom Schmelzpunkt 207°;

f) aus 9-Acridanon und 2-(4-Morpholinyl)-äthylchlorid-hydrochlorid das 10-[2-(4-Morpholinyl)äthyl]-9-acridanon vom Schmelzpunkt 196° und daraus das

10-[2-(4-Morpholinyl)äthyl]-9-acridanon-(2-thiazolidinyliden)hydrazon

vom Schmelzpunkt 236°.

Beispiel 2

a) Man rührt eine Mischung aus 4,5 g 9-Acridanon, 1,1 g Natriumhydrid und 100 ml Dimethylformamid während 0,5 Stunden, versetzt dann mit 5,2 g

2-(4-Acetyl-1-piperazinyl)-äthylchlorid-hydrochlorid,

rührt während 15 Stunden bei 70° und dampft ein. Man versetzt den Rückstand mit Wasser, extrahiert mit Methylenchlorid, wäscht den Auszug mit Wasser, trocknet diesen über Natriumsulfat und dampft ein. Durch Kristallisieren aus Äthanol erhält man

10-[2-(4-Acetyl-1-piperazinyl)äthyl]-9-acridanon

vom Schmelzpunkt 241–243°.

Man versetzt eine Suspension von 3,5 g (10 mMol) 10-[2-(4-Acetyl-1-piperazinyl)-äthyl]-9-acridanon

in 100 ml Acetonitril mit 1,7 ml (20 mMol) Oxalylchlorid, nutscht nach 0,5 Stunden ab und wäscht das abgenutschte Material

(9-Chlor-10-[2-(4-acetyl-1-piperazinyl)-äthyl]acridiniumchlorid)

nacheinander mit Acetonitril und Äther. Man erhitzt eine Mischung des erhaltenen Stoffes mit 2 g (10 mMol) 2-Hydrazino-2-thiazolin-hydrobromid, 2,5 g (30 mMol) Natriumacetat und 100 ml Methanol unter Rückfluss zum Sieden, dampft nach 15 Minuten ein, versetzt den Rückstand mit Wasser, stellt mit Sodalösung alkalisch und extrahiert mit Methylenchlorid. Der Auszug wird mit Wasser gewaschen, getrocknet und eingedampft, worauf man den Rückstand aus Methanol kristallisiert. Nach Umkristallisieren aus n-Butanol erhält man

10-[2-(4-Acetyl-1-piperazinyl)äthyl]-9-acridanon-(2-thiazolidinyliden)hydrazon

vom Schmelzpunkt 256°.

In analoger Weise erhält man:

b) Aus 9-Acridanon und 2-(4-Äthoxycarbonyl-1-piperazinyl)äthylchlorid-hydrochlorid das 10-[2-(4-Äthoxycarbonyl-1-piperazinyl)-äthyl]-9-acridanon

vom Schmelzpunkt 147° und daraus das

10-[2-(4-Äthoxycarbonyl-1-piperazinyl)-äthyl]-9-acridanon-(2-thiazolidinyliden)-hydrazon

vom Schmelzpunkt 231° (Zersetzung);

c) aus 9-Acridanon und 2-(4-Pivaloyl-1-piperazinyl)-äthylchlorid-hydrochlorid das

10-[2-(4-Pivaloyl-1-piperazinyl)äthyl]-9-acridanon

vom Schmelzpunkt 184° und daraus das

10-[2-(4-Pivaloyl-1-piperazinyl)äthyl]-9-acridanon-(2-thiazolidinyliden)hydrazon

vom Schmelzpunkt 208°;

d) aus 9-Acridanon und

2-[4-(Methylsulphonyl)-1-piperazinyl]äthylchlorid-hydrochlorid

das

10-[2-[4-(Methylsulphonyl)-1-piperazinyl]-äthyl]-9-acridanon

vom Schmelzpunkt 244–246° und daraus das

10-[2-[4-(Methylsulphonyl)-1-piperazinyl]-äthyl]-9-acridanon-(2-thiazolidinyliden)-hydrazon

vom Schmelzpunkt 223–235° (Zersetzung).

Beispiel 3

a) Man rührt eine Mischung aus 3,9 g Acridanon, 1,0 g Natriumhydrid und 80 ml Dimethylformamid während 0,5 Stunden, versetzt dann mit 2,9 g 1-Chlor-2-dimethylaminoäthan-hydrochlorid, rührt während 18 Stunden bei 60°, dampft ein und extrahiert den Rückstand mit Methylenchlorid. Man wäscht den Auszug mit Wasser, trocknet über Natriumsulfat und dampft ein. Durch Kristallisieren aus Isopropyläther erhält man 10-[2-(Dimethylamino)äthyl]-9-acridanon vom Schmelzpunkt 145–146°.

Man rührt eine Lösung von 3 g 10-[2-(Dimethylamino)äthyl]-9-acridanon in 100 ml Dichlormethan und 1,93 ml Oxalylchlorid während 0,5 Stunden. Nach Eindampfen wird der Rückstand mit 2,25 g 2-Hydrazino-2-thiazolin-hydrobromid und 3 g Natriumacetat in 100 ml Methanol gerührt, worauf man 10 Minuten unter Rückfluss zum Sieden erhitzt und erneut eindampft. Man versetzt den Rückstand mit Wasser, stellt mit Sodalösung alkalisch und extrahiert mit Methylenchlorid. Der Auszug wird mit Wasser gewaschen, über Natriumsulfat getrocknet und eingedampft. Nach Kristallisieren aus Acetonitril erhält man
10-[2-(Dimethylamino)äthyl]-9-acridanon-(2-thiazolidinyliden)hydrazon
vom Schmelzpunkt 192–194°.

In analoger Weise erhält man:

b) Aus 9-Acridanon und 2-(Dimethylamino)propylchlorid-hydrochlorid das 10-[2-(Dimethylamino)propyl]-9-acridanon vom Schmelzpunkt 114° und daraus das
10-[2-(Dimethylamino)propyl]-9-acridanon-(2-thiazolidinyliden)hydrazon
vom Schmelzpunkt 152°;

c) aus 9-Acridanon und 2-(2,6-Dimethyl-1-piperidinyl)äthylchlorid-hydrochlorid das 10-[2-(2,6-Dimethyl-1-piperidinyl)äthyl]-9-acridanon vom Schmelzpunkt 146° und daraus das
10-[2-(2,6-Dimethyl-1-piperidinyl)äthyl]-9-acridanon-(2-thiazolidinyliden)hydrazon
vom Schmelzpunkt 161°;

d) aus 9-Acridanon und (1-Methyl-4-imidazolyl)-methylchlorid-hydrochlorid das 10-[(1-Methyl-4-imidazolyl)methyl]-9-acridanon vom Schmelzpunkt 205–207° und daraus das
10-[(1-Methyl-4-imidazolyl)methyl]-9-acridanon-(2-thiazolidinyliden)hydrazon
vom Schmelzpunkt 206–208°;

e) aus 9-Acridanon und
2-[4-[2-(Dimethylamino)acetyl]-1-piperazinyl]-äthylchlorid-hydrochlorid
das
10-[2-[4-[2-(Dimethylamino)acetyl]-1-piperazinyl]äthyl]-9-acridanon
vom Schmelzpunkt 146–147° und daraus das
10-[2-[4-[2-(Dimethylamino)acetyl]-1-piperazinyl]äthyl]-9-acridanon-(2-thiazolidinyliden)hydrazon
vom Schmelzpunkt 129–131°;

f) aus 1-Chlor-9-acridanon und 2-(Diäthylamino)äthylchlorid-hydrochlorid das 10-[2-(Diäthylamino)äthyl]-1-chlor-9-acridanon vom Schmelzpunkt 121° und daraus das
10-[2-(Diäthylamino)äthyl]-1-chlor-9-acridanon-(2-thiazolidinyliden)hydrazon
vom Schmelzpunkt 181–183°;

g) aus 9-Acridanon und 2-(4-Propyl-1-piperazinyl)äthylchlorid-dihydrochlorid das 10-[2-(4-Propyl-1-piperazinyl)äthyl]-9-acridanon und daraus das 10-[2-(4-Propyl-1-piperazinyl)äthyl]-9-acridanon-(2-thiazolidinyliden)hydrazon
vom Schmelzpunkt 205–207° (Zersetzung).

Beispiel 4

Man rührt eine Lösung von 4,94 g (16,8 mMol) 10-[2-(Diäthylamino)äthyl]-9-acridanon in 300 ml Dichlormethan mit 2,85 ml (33,6 mMol) Oxalylchlorid während 1 Stunde. Das entstandene Salz
(9-Chlor-10-[2-(diäthylamino)äthyl]-acridiniumchlorid
wird abfiltriert und mit 100 ml Methanol, 2,82 g (16,8 mMol) 2-(3-Methyl)thiazolidinyliden-hydrazin-hydrochlorid und 4,1 g (50 mMol) Natriumacetat versetzt. Man erhitzt während 10 Minuten unter Rückfluss zum Sieden, dampft ein, versetzt den Rückstand mit Wasser, stellt mit Sodalösung alkalisch und extrahiert mit Dichlormethan. Der Auszug wird mit Wasser gewaschen, getrocknet und eingedampft. Durch Kristallisieren aus Äthanol erhält man
10-[2-(Diäthylamino)äthyl]-9-acridanon-(3-methyl-2-thiazolidinyliden)hydrazon
vom Schmelzpunkt 123–125°.

Beispiel 5

a) Man versetzt eine Lösung von 11,5 g 10-[2-(Diäthylamino)äthyl]-9-acridanon in 250 ml Dichlormethan bei −5° und innerhalb 0,5 Stunden mit 6,7 ml Oxalylchlorid, rührt während 1 Stunde bei Raumtemperatur, filtriert die gelben Kristalle
(9-Chlor-10-[2-(diäthylamino)äthyl]-acridiniumchlorid),
die sich gebildet haben ab und wäscht diese nacheinander mit Methylenchlorid und Petroläther. Man nimmt das erhaltene Material in 200 ml Methanol auf, versetzt mit 3,6 g Thiosemicarbazid, rührt während etwa 18 Stunden bei Raumtemperatur, filtriert das ausgefallene Salz ab und wäscht es nacheinander mit Äthanol und Äther. Man erhält
10-[2-(Diäthylamino)äthyl]-9-acridanon-thiosemicarbazon-dihydrochlorid
(kristallisiert mit 1 Mol Methanol) vom Schmelzpunkt 124° (Zersetzung), versetzt dieses mit 1N-Natronlauge und extrahiert mit Dichlormethan. Die Lösung wird mit Wasser gewaschen, getrocknet und eingedampft, worauf das Produkt aus Acetonitril kristallisiert wird. Man erhält
10-[2-(Diäthylamino)äthyl]-9-acridanon-thiosemicarbazon
vom Schmelzpunkt 154–156°.

Eine Lösung von 6,1 g 10-[2-(Diäthylamino)-äthyl]-9-acridanon-thiosemicarbazon in 100 ml Dimethylformamid wird während etwa 18 Stunden mit 4,3 ml Chloracetaldehyd (50-proz. Lösung in Wasser) gerührt. Anschliessend dampft man ein, nimmt den Rückstand in 50 ml Äthanol auf, stellt mit äthanolischer Salzsäure kongosauer und filtriert die gebildeten Kristalle ab. Man wäscht nacheinander mit Äthanol, Äther und Petroläther und erhält 10-[2-(Diäthylamino)äthyl]-9-acridanon-(2-thiazolyl)hydrazon-dihydrochlorid vom Schmelzpunkt 214–216° (Zersetzung).

In analoger Weise erhält man:

b) Aus 10-[2-(1-Piperidinyl)äthyl]-9-acridanon und Thiosemicarbazid das
10-[2-(1-Piperidinyl)äthyl]-9-acridanon-thiosemicarbazon

vom Schmelzpunkt 187° (Zersetzung) und daraus das
10-[2-(1-Piperidinyl)äthyl]-9-acridanon-(2-thiazolyl)hydrazon-1,5 HCl
vom Schmelzpunkt 190° (Zersetzung);

c) aus 10-[2-(4-Morpholinyl)äthyl]-9-acridanon und Thiosemicarbazid das
10-[2-(4-Morpholinyl)äthyl]-9-acridanon-thiosemicarbazon
vom Schmelzpunkt 240° (Zersetzung) und daraus das
10-[2-(4-Morpholinyl)äthyl]-9-acridanon-(2-thiazolyl)hydrazon-dihydrochlorid
vom Schmelzpunkt 225° (Zersetzung);

d) aus
10-[2-(4-Acetyl-1-piperazinyl)äthyl]-9-acridanon
und Thiosemicarbazid das
10-[2-(4-Acetyl-1-piperazinyl)äthyl]-9-acridanon-thiosemicarbazon
vom Schmelzpunkt 225° (Zersetzung) und daraus das
10-[2-(4-Acetyl-1-piperazinyl)äthyl]-9-acridanon-(2-thiazolyl)hydrazon-dihydrochlorid
vom Schmelzpunkt 180° (Zersetzung);

e) aus 10-[2-(4-Pivaloyl-1-piperazinyl)äthyl]-9-acridanon und Thiosemicarbazid das
10-[2-(4-Pivaloyl-1-piperazinyl)äthyl]-9-acridanon-thiosemicarbazon
vom Schmelzpunkt 179° (Zersetzung) und daraus das
10-[2-(4-Pivaloyl-1-piperazinyl)äthyl]-9-acridanon-(2-thiazolyl)hydrazon-dihydrochlorid
vom Schmelzpunkt 210° (Zersetzung);

f) aus 10-[3-(Dimethylamino)propyl]-9-acridanon und Thiosemicarbazid das
10-[3-(Dimethylamino)propyl]-9-acridanon-thiosemicarbazon
vom Schmelzpunkt 202–204° und daraus das
10-[3-(Dimethylamino)propyl]-9-acridanon-(2-thiazolyl)hydrazon-dihydrochlorid
vom Schmelzpunkt 195° (Zersetzung).

Beispiel 6

a) Man rührt eine Mischung aus 3 g (9,33 mMol) 10-[2-(4-Methyl-1-piperazinyl)äthyl]-9-acridanon, 100 ml Dichlormethan und 1,6 ml (18,66 mMol) Oxalylchlorid während 0,5 Stunden, engt auf das halbe Volumen ein und fällt das Salz
(9-Chlor-10-[2-(4-methyl-1-piperazinyl)äthyl]-acridiniumchlorid)
durch Zugabe von 100 ml Essigester aus. Es wird abfiltriert, mit Petroläther gewaschen und getrocknet. Das rotbraune Pulver erhitzt man zusammen mit 1,42 g (9,33 mMol) 2-Thiazolyl-hydrazin-hydrochlorid, 2,3 g (28 mMol) Natriumacetat und 100 ml Methanol während 5 Minuten unter Rückfluss zum Sieden, dampft ein, versetzt den Rückstand mit 50 ml Wasser, stellt mit Sodalösung alkalisch und extrahiert mit Dichlormethan. Der Auszug wird mit 10-proz. Kochsalzlösung gewaschen, getrocknet und eingedampft. Den Rückstand nimmt man in 100 ml Methanol auf, säuert mit äthanolischer Salzsäure an, filtriert das ausgefallene Produkt ab und wäscht es nacheinander mit wenig kaltem Äthanol und Petroläther. Man erhält
10-[2-(4-Methyl-1-piperazinyl)äthyl]-9-acridanon-(2-thiazolyl)hydrazon.2,5
HCl vom Schmelzpunkt 230° (Zersetzung).

In analoger Weise erhält man:

b) Aus 10-[2-(Dimethylamino)äthyl]-9-acridanon und 2-Thiazolyl-hydrazin-hydrochlorid das
10-[2-(Dimethylamino)äthyl]-9-acridanon-(2-thiazolyl)hydrazon-dihydrochlorid
vom Schmelzpunkt >200° (Zersetzung);

c) aus 10-[2-(4-Äthoxycarbonyl-1-piperazinyl)äthyl]-9-acridanon und 2-Thiazolyl-hydrazin-hydrochlorid das
10-[2-(4-Äthoxycarbonyl-1-piperazinyl)-äthyl]-9-acridanon-(2-thiazolyl)hydrazon-dihydrochlorid
vom Schmelzpunkt 185° (Zersetzung);

d) aus 10-[2-[4-(Methylsulphonyl)-1-piperazinyl]äthyl]-9-acridanon und 2-Thiazolyl-hydrazin-hydrochlorid das
10-[2-[4-(Methylsulphonyl)-1-piperazinyl]-äthyl]-9-acridanon-(2-thiazolyl)hydrazon-dihydrochlorid vom Schmelzpunkt 200°;

e) aus 10-[2-(4-Propyl-1-piperazinyl)äthyl]-9-acridanon und 2-Thiazolyl-hydrazin-hydrochlorid das
10-[2-(4-Propyl-1-piperazinyl)äthyl]-9-acridanon-(2-thiazolyl)hydrazon.2,5 HCl
vom Schmelzpunkt 220° (Zersetzung).

Beispiel 7

a) Man rührt eine Mischung aus 9,75 g (50 mMol) 9-Acridanon, 200 ml Dimethylformamid und 4,8 g (200 mMol) Natriumhydrid während 0,5 Stunden, versetzt portionenweise mit 17,8 g (50 mMol)
2-[4-[2-(Diäthylamino)äthyl]-1-piperazinyl]-äthylchlorid-trihydrochlorid,
rührt während 18 Stunden bei 70°, erhitzt dann während 8 Stunden unter Rückfluss zum Sieden und dampft ein. Man versetzt den Rückstand mit 100 ml Wasser, extrahiert mit Dichlormethan, wäscht den Auszug mit Wasser, trocknet diesen und dampft ein. Das Produkt wird zweimal aus Petroläther kristallisiert und man erhält
10-[2-[4-[2-(Diäthylamino)äthyl]-1-piperazinyl]-äthyl-9-acridanon
vom Schmelzpunkt 106–108°.

Man versetzt eine Suspension von 4,06 g (10 mMol) dieses Stoffes in 100 ml trockenem Acetonitril unter Rühren portionenweise mit 1,7 ml (20 mMol) Oxalylchlorid, dampft nach 0,5 Stunden ein, versetzt den Rückstand mit Acetonitril, nutscht ab, wäscht das erhaltene Material nacheinander mit Äther und Petroläther und trocknet es. Man nimmt das braune Pulver
(9-Chlor-10-[2-[4-[2-(diäthylamino)äthyl]-1-piperazinyl]äthyl]acridinium-chlorid)
in 100 ml Methanol auf, versetzt mit 1,52 g (10 mMol) 2-Thiazolyl-hydrazin-hydrochlorid und 2,46 g (30 mMol) Natriumacetat und erhitzt während 10 Minuten unter Rückfluss zum Sieden. Nach dem Eindampfen versetzt man den Rückstand mit 100 ml Wasser, stellt mit 2N-Natronlauge

phenolphthaleinalkalisch, nimmt das ausgefallene Produkt in 100 ml Dichlormethan auf, wäscht die Lösung mit Wasser neutral, trocknet sie und dampft ein. Der Rückstand wird in 100 ml Methanol gelöst, worauf man mit ätherischer Salzsäure ansäuert, das ausgefallene Salz abfiltriert und nacheinander mit Methanol, Äther und Petroläther wäscht. Man erhält

10-[2-[4-[2-(Diäthylamino)äthyl]-1-piperazinyl]äthyl]-9-acridanon-(2-thiazolyl)-hydrazon.3,5 HCl

vom Schmelzpunkt 235° (Zersetzung).

In analoger Weise erhält man:

b) Aus 10-[2-[4-[2-(Diäthylamino)äthyl]-1-piperazinyl]äthyl]-9-acridanon und 2-Hydrazino-2-thiazolin-hydrobromid das

10-[2-[4-[2-(Diäthylamino)äthyl]-1-piperazinyl]äthyl]-9-acridanon-(2-thiazolidinyliden)hydrazon

vom Schmelzpunkt 144–146° (Zersetzung).

Beispiel 8

a) Man rührt eine Mischung aus 4,94 g (16,8 mMol) 10-[2-(Diäthylamino)äthyl]-9-acridanon, 300 ml Dichlormethan und 2,85 ml (33,6 mMol) Oxalylchlorid während 20 Minuten, filtriert die erhaltenen Kristalle

(9-Chlor-10-[2-(diäthylamino)äthyl]-acridinium-chlorid)

und wäscht sie mit Äther. Man nimmt dieses Material in 100 ml Methanol auf, versetzt mit 2,8 g (16,8 mMol) N-Methyl-N-(2-thiazolyl)hydrazin-hydrochlorid und 4,1 g (50 mMol) Natriumacetat, erhitzt während 15 Minuten unter Rückfluss zum Sieden und dampft ein. Der Rückstand wird mit Sodalösung alkalisch gestellt, worauf man mit Dichlormethan extrahiert, den Auszug mit Wasser wäscht, trocknet und eindampft. Durch Kristallisieren des Rückstandes aus Isopropyläther erhält man

10-[2-(Diäthylamino)äthyl]-9-acridanon-methyl(2-thiazolyl)hydrazon

vom Schmelzpunkt 103–104°.

In analoger Weise erhält man:

b) Aus 10-[2-(Diäthylamino)äthyl]-9-acridanon und N-Methyl-N-(2-thiazolin-2-yl)hydrazon das

10-[2-(Diäthylamino)äthyl]-9-acridanon-methyl(2-thiazolin-2-yl)hydrazon

vom Schmelzpunkt 109–110°.

Beispiel 9

Man versetzt eine unter Rückfluss zum Sieden erhitzte Suspension von 58,6 g (0,3 Mol) 9-Acridanon in 200 ml Dimethylformamid portionenweise mit insgesamt 60 g (0,33 Mol) Äthylencarbaminsäurebenzylester. Nach 53 Stunden lässt man abkühlen, filtriert das kristalline Produkt ab und wäscht es nacheinander mit Dimethylformamid, Aceton und Äther. Die Mutterlauge wird mit Wasser versetzt, worauf das ausgefallene Produkt abfiltriert und wie oben gewaschen wird. Das vereinigte Material wird aus Äthanol kristallisiert und man erhält

[2-(9-Oxo-10(9H)-acridinyl)äthyl]carbaminsäurebenzylester vom Schmelzpunkt 224–226°.

9,31 g (25 mMol) dieses Stoffes werden in 100 ml Dichlormethan suspendiert. Man versetzt mit 4,5 ml (50 mMol) Oxalylchlorid, rührt während 1 Stunde und dampft die Lösung ein. Den Rückstand nimmt man in 400 ml Acetonitril auf, versetzt mit 5,55 g (28 mMol) 2-Hydrazino-2-thiazolin-hydrobromid und erhitzt während 24 Stunden unter Rückfluss zum Sieden. Man filtriert das noch warme Gemisch, wäscht das erhaltene Material nacheinander mit Acetonitril und Äther, versetzt es mit Methylenchlorid und Wasser und setzt durch Zugabe von Sodalösung die Base frei. Man reinigt die freie Base durch Chromatographie an Kieselgel unter Eluieren mit Dichlormethan und überführt sie mit äthanolischer Salzsäure ins Hydrochlorid. Durch Zugabe von Äther zur erhaltenen Lösung erhält man kristallines

[2-[9-(2-Thiazolidinylidenhydrazono)-10-acridanyl]äthyl]carbaminsäurebenzylester-dihydrochlorid

vom Schmelzpunkt 167,7–170,8° (Zersetzung).

Eine Suspension von 3,5 g (6,4 mMol) des obigen Stoffes in 300 ml Eisessig wird mit 10 ml bromwasserstoffhaltigem Eisessig (etwa 30-proz.) während 3 Tagen gerührt, worauf das Produkt abfiltriert und mit Eisessig und Äther gewaschen wird. Anschliessend nimmt man es in Wasser auf, setzt mit Soda die Base frei und nimmt diese in Methylenchlorid auf. Die Methylenchloridlösung wird über Natriumsulfat getrocknet und eingedampft. Den Rückstand löst man in chlorwasserstoff-haltigem Äthanol und kristallisiert das Salz durch Zugabe von Äther aus. Man erhält

10-(2-Aminoäthyl)-9-acridanon-(2-thiazolidinyliden)hydrazon-dihydrochlorid

vom Schmelzpunkt 226–231°.

Beispiel 10

Man suspendiert 2,25 g (6 mMol)

[2-(9-Oxo-10(9H)-acridinyl)äthyl]carbaminsäurebenzylester

in 20 ml Methylenchlorid und versetzt mit 1,05 ml (12 mMol) Oxalylchlorid. Nach 1 Stunde wird die Lösung eingedampft, worauf man den Rückstand in 200 ml Acetonitril aufnimmt, mit 1,09 g (7,2 mMol) 2-Thiazolyl-hydrazin-hydrochlorid versetzt und während 1,5 Stunden unter Rückfluss zum Sieden erhitzt. Man lässt abkühlen, nutscht die Kristalle ab und wäscht sie mit Acetonitril und Äther. Man erhält

[2-[9-(2-Thiazolylhydrazono)-9-acridanyl]-äthyl]carbaminsäurebenzylester-hydrochlorid

vom Schmelzpunkt 207–209° (Zersetzung).

Eine Suspension von 7,6 g (15 mMol) dieses Stoffes in 450 ml Eisessig wird mit 20 ml bromwasserstoff-haltigem Eisessig (etwa 30-proz.) versetzt und während 2 Tagen gerührt. Das Produkt wird abgesaugt und mit Eisessig und Äther gewaschen. Mit einem salzsauren Ionenaustauscher wird das Salzgemisch ins Hydrochlorid übergeführt. Die wässrige Lösung wird gefriergetrocknet, und der kristalline Rückstand wiederholt aus Methanol/Äther umgelöst. Man erhält

10-(2-Aminoäthyl)-9-acridanon-2-thiazolyl-
hydrazon-dihydrochlorid
vom Schmelzpunkt 204–214° (Zersetzung).

Beispiel 11

Eine Suspension von 11,2 g (30 mMol) [2-(9-Oxo-10(9H)-acridinyl)äthyl]carbaminsäurebenzylester in 100 ml Eisessig wird mit 20 ml bromwasserstoff-haltigem Eisessig (etwa 30-proz.) versetzt und während 1 Stunde gerührt. Das Produkt wird abfiltriert und mit Eisessig und Äther gewaschen. Man erhält 10-(2-Aminoäthyl)-9-acridanon-hydrobromid vom Schmelzpunkt >250°. Die freie Base wird aus einer wässrigen Lösung mit Natriumbicarbonatlösung ausgefällt, abfiltriert und mit Wasser und Aceton gewaschen. Das daraus gewonnene und analysierte Hydrochlorid schmilzt über 250°.

6,11 g (27 mMol) der obigen freien Base werden in 100 ml Pyridin suspendiert, worauf man mit 2,33 ml (30 mMol) Methansulfochlorid versetzt. Unter Erwärmung entsteht eine klare Lösung, welcher man nach 3 Stunden 700 ml Wasser zufügt. Allmählich bilden sich Kristalle. Man konzentriert auf 300 ml, fügt 500 ml Wasser dazu und konzentriert im Vakuum wieder auf 300 ml. Das Produkt wird abfiltriert, mit Wasser gewaschen und aus Äthanol umkristallisiert. Man erhält
N-[2-(9-Oxo-10-acridanyl)äthyl]methansulfon-
amid
vom Schmelzpunkt 226–227°.

950 mg (3 mMol) dieser Substanz werden in 10 ml Methylenchlorid suspendiert, worauf man mit 0,8 ml (9 mMol) Oxalylchlorid versetzt. Nach 1 Stunde wird eingedampft. Den Rückstand nimmt man in 100 ml Acetonitril auf und versetzt mit 713 mg (3,6 mMol) 2-Hydrazino-2-thiazolin-hydrobromid. Nach 20 Minuten unter Rückfluss wird das Produkt abfiltriert, mit Äther gewaschen und in 150 ml Methylenchlorid/Methanol (97:3) gelöst. Man stellt mit Natriumbicarbonatlösung alkalisch, extrahiert mit Methylenchlorid, trocknet über Natriumsulfat und dampft ein. Nach Kristallisieren aus Methylenchlorid/Methanol/Petroläther erhält man
N-[2-[9-(2-Thiazolin-2-yl)hydrazono]-10-
acridanyl]äthyl]methansulfonamid
vom Schmelzpunkt 206–207° (Zersetzung).

Beispiel 12

a) Eine Suspension von 1,44 g (60 mMol) Natriumhydrid in 100 ml Dimethylformamid wird innert 10 Minuten mit 9,75 g (50 mMol) 9-Acridanon versetzt und 2 Stunden bei 50° gehalten. Bei 120° werden dann 9,5 g (53 mMol) 4,4-Diäthoxybutylchlorid zugegeben. Nach 16 Stunden kühlt man ab, giesst das Gemisch auf Wasser, extrahiert mit Dichlormethan, trocknet den Auszug über Natriumsulfat und dampft ein. Der Rückstand wird mit Hexan extrahiert und zweimal aus Hexan kristallisiert. Man erhält 10-(4,4-Diäthoxybutyl)-9-acridanon vom Schmelzpunkt 73–74,5°.

8,5 g (25 mMol) dieses Stoffes werden in 20 ml Diäthylamin gelöst, worauf man mit 15 ml Ameisensäure versetzt, während 20 Stunden auf 100° erhitzt, abkühlt, Wasser und verdünnte Salzsäure zugibt und die wässrige Lösung mit Äther wäscht. Die wässrige Lösung wird mit Natronlauge versetzt und mit Methylenchlorid extrahiert. Der Auszug wird über Natriumsulfat getrocknet und eingedampft. Der Rückstand wird aus Hexan kristallisiert. Man erhält 10-[4-(Diäthylamino]butyl]-9-acridanon vom Schmelzpunkt 66,5–68,7°. Das Hydrochlorid hat einen Schmelzpunkt von 126,8–128,3°.

Zu einer Lösung von 3,0 g (9,3 mMol) der obigen freien Base in 200 ml Dichlormethan werden bei 0° innerhalb 3 Minuten 2,4 ml (27,9 mMol) Oxalylchlorid getropft. Man rührt während 1 Stunde bei Raumtemperatur, dampft ein, nimmt den Rückstand
(9-Chlor-10-[4-(diäthylamino)butyl]-
acridinium-chlorid
in 300 ml Acetonitril auf, versetzt mit 1,57 g (10,2 mMol) 2-Hydrazino-2-thiazolin-hydrochlorid, erhitzt während 0,5 Stunden unter Rückfluss zum Sieden, rührt noch 1,5 Stunden bei Raumtemperatur und kühlt auf 0° ab. Das Produkt wird abfiltriert, mit Acetonitril und Äther gewaschen und in Wasser aufgenommen. Man stellt die Lösung mit Soda alkalisch, extrahiert mit Methylenchlorid, trocknet die Methylenchloridlösung über Natriumsulfat und dampft ein. Durch Kristallisieren aus Dichlormethan/Äther/Petroläther erhält man
10-[4-(Diäthylamino)butyl]-9-acridanon-(2-
thiazolidinyliden)hydrazon
vom Schmelzpunkt 105,6–107,2°.

In analoger Weise erhält man:

b) Aus 9-Acridanon und 5,5-Diäthoxypentylchlorid das 10-(5,5-Diäthoxypentyl)-9-acridanon vom Schmelzpunkt 82–83°, daraus das 10-[5-(Diäthylamino)pentyl]-9-acridanon vom Schmelzpunkt 59–61,2° und daraus das
10-[5-(Diäthylamino)pentyl]-9-acridanon-(2-
thiazolidinyliden)hydrazon
vom Schmelzpunkt 128,5–130,1°.

Beispiel A

Herstellung von Tabletten nachstehender Zusammensetzung:

| | mg/Tablette |
|---|---|
| Wirkstoff | 100 |
| Milchzucker | 100 |
| Maisstärke | 85 |
| Povidon | 10 |
| Talk | 3 |
| Magnesiumstearat | 2 |
| Tablettengewicht | 300 mg |

Man vermischt den Wirkstoff mit dem Milchzukker und der Maisstärke, befeuchtet mit einer wässrigen Lösung von Povidon und granuliert. Das Granulat wird bei 40° getrocknet und gesiebt. Man vermischt mit dem Talk und dem Magnesiumstearat und verpresst zu Tabletten.

Die folgenden Verbindungen der Formel I können wie oben beschrieben als Wirkstoffe verarbeitet werden:
10-[2-(4-Methyl-1-piperazinyl)äthyl-9-acridanon-(2-thiazolyl)hydrazon,
10-[2-(4-Methyl-1-piperazinyl)äthyl]-9-acridanon-(2-thiazolidinyliden)hydrazon,
10-[2-(4-Acetyl-1-piperazinyl)äthyl]-9-acridanon-(2-thiazolidinyliden)hydrazon,
10-[2-(Dimethylamino)äthyl]-9-acridanon-(2-thiazolidinyliden)hydrazon,
10-[2-(Diäthylamino)äthyl]-9-acridanon-(2-thiazolyl)hydrazon,
10-[2-(1-Piperidinyl)äthyl]-9-acridanon-(2-thiazolyl)hydrazon,
10-(2-Aminoäthyl)-9-acridanon-2-(thiazolyl)-hydrazon,
10-[2-(Dimethylamino)äthyl]-9-acridanon-(2-thiazolyl)hydrazon,
10-[2-(4-Acetyl-1-piperazinyl)äthyl]-9-acridanon-(2-thiazolyl)hydrazon,
10-[3-(Dimethylamino)propyl]-9-acridanon-(2-thiazolyl)hydrazon,
10-[2-(Diäthylamino)äthyl]-9-acridanon-(2-thiazolidinyliden)hydrazon,
10-[2-(1-Pyrrolidinyl)äthyl]-9-acridanon-(2-thiazolidinyliden)hydrazon und
10-[3-(Dimethylamino)propyl]-9-acridanon-(2-thiazolidinyliden)hydrazon.

**Patentansprüche für die Vertragsstaaten BE, CH, DE, FR, GB, IT, LI, LU, NL, SE**

1. Acridanon-Derivate der allgemeinen Formel

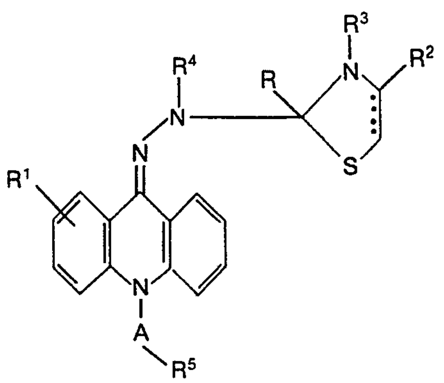

I

worin die punktierte Linie eine fakultative Bindung, $R^1$ Wasserstoff, Halogen oder Nitro, $R^2$ Wasserstoff oder niederes Alkyl, eines der Symbole $R^3$ und $R^4$ Wasserstoff oder niederes Alkyl und das andere zusammen mit R eine zusätzliche Bindung, A niederes Alkylen, $R^5$ einen 5-gliedrigen, stickstoffhaltigen, aromatischen, gegebenenfalls durch niederes Alkyl substituierten Heterocyclus, Amino oder eine Gruppe

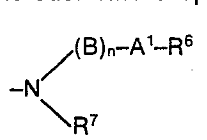

oder

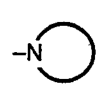

,

das Symbol

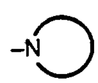

einen 5- oder 6-gliedrigen, gegebenenfalls durch niederes Alkyl substituierten gesättigten Heterocyclus, der als Ringglied noch ein Sauerstoff- oder Schwefelatom oder die Gruppe >NH oder $>N(B)_n-A^1-R^6$ enthalten kann, B die Gruppe –CO–, –COO– oder $-SO_2-$, n die Zahl 0 oder 1, $A^1$ niederes Alkylen, $R^6$ Wasserstoff, Amino, niederes Alkylamino oder Di(niederes Alkyl)amino und $R^7$ Wasserstoff oder niederes Alkyl bedeuten, und die als nieder bezeichneten Reste 1 bis 7 Kohlenstoffatome enthalten, und pharmazeutisch annehmbare Säureadditionssalze davon.

2. Verbindungen gemäss Anspruch 1, worin ein allfälliger Substituent $R^1$ sich in der 1-Stellung befindet.

3. Verbindungen gemäss Anspruch 1 oder 2, worin $R^1$ Wasserstoff bedeutet.

4. Verbindungen gemäss einem der Ansprüche 1 bis 3, worin $R^2$ Wasserstoff bedeutet.

5. Verbindungen gemäss einem der Ansprüche 1 bis 4, worin eines der Symbole $R^3$ und $R^4$ Wasserstoff und das andere zusammen mit R eine zusätzliche Bindung bedeuten.

6. Verbindungen gemäss einem der Ansprüche 1 bis 5, worin A Dimethylen oder Trimethylen bedeutet.

7. Verbindungen gemäss einem der Ansprüche 1 bis 6, worin $R^5$ Amino, 1-Pyrrolidinyl, 1-Piperidinyl, 1-Morpholinyl, 1-Piperazinyl oder die Gruppe $-NR^7-A^1-R^{61}$ oder

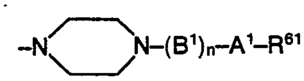

,

$A^1$ niederes Alkylen, $B^1$ die Gruppe –CO–, n die Zahl 0 oder 1, $R^{61}$ Wasserstoff und $R^7$ Wasserstoff oder niederes Alkyl bedeuten.

8. Verbindungen gemäss Anspruch 7, worin $R^5$ Amino, Dimethylamino, Diäthylamino, 1-Pyrrolidinyl, 1-Piperidinyl, 4-Methyl-1-piperazinyl oder 4-Acetyl-1-piperazinyl bedeutet.

9. 10-[2-(4-Methyl-1-piperazinyl)äthyl]-9-acridanon-(2-thiazolyl)hydrazon.

10. 10-[2-(4-Methyl-1-piperazinyl)äthyl]-9-acridanon-(2-thiazolidinyliden)hydrazon.

11. 10-[2-(4-Acetyl-1-piperazinyl)äthyl]-9-acridanon-(2-thiazolidinyliden)hydrazon.

12. 10-[2-(Dimethylamino)äthyl]-9-acridanon-(2-thiazolidinyliden)hydrazon.

13. 10-[2-(Diäthylamino)äthyl]-9-acridanon-(2-thiazolyl)hydrazon.

14. 10-[2-(1-Piperidinyl)äthyl]-9-acridanon-(2-thiazolyl)hydrazon.

15. 10-(2-Aminoäthyl)-9-acridanon-(2-thiazolyl)-hydrazon.

16. 10-[2-(Dimethylamino)äthyl]-9-acridanon-(2-thiazolyl)hydrazon.

17. 10-[2-(4-Acetyl-1-piperazinyl)äthyl]-9-acridanon-(2-thiazolyl)hydrazon.

18. 10-[3-(Dimethylamino)propyl]-9-acridanon-(2-thiazolyl)hydrazon.

19. 10-[2-(Diäthylamino)äthyl]-9-acridanon-(2-thiazolidinyliden)hydrazon.

20. 10-[2-(1-Pyrrolidinyl)äthyl]-9-acridanon-(2-thiazolidinyliden)hydrazon.

21. 10-[3-(Dimethylamino)propyl]-9-acridanon-(2-thiazolidinyliden)hydrazon.

22. 10-[2-(4-Propyl-1-piperazinyl)äthyl]-9-acridanon-(2-thiazolidinyliden)hydrazon,
10-[2-(Dimethylamino)propyl]-9-acridanon-(2-thiazolidinyliden)hydrazon,
10-[2-(1-Piperidinyl)äthyl]-9-acridanon-(2-thiazolidinyliden)hydrazon,
10-[2-(2,6-Dimethyl-1-piperidinyl)äthyl]-9-acridanon-(2-thiazolidinyliden)hydrazon,
10-[2-(4-Morpholinyl)äthyl]-9-acridanon-(2-thiazolidinyliden)hydrazon,
10-[2-(4-Äthoxycarbonyl-1-piperazinyl)-äthyl]-9-acridanon-(2-thiazolidinyliden)-hydrazon,
10-[2-(4-Pivaloyl-1-piperazinyl)äthyl]-9-acridanon-(2-thiazolidinyliden)hydrazon,
10-[2-[4-(Methylsulfonyl)-1-piperazinyl]-äthyl]-9-acridanon-(2-thiazolidinyliden)-hydrazon,
10-[(1-Methyl-4-imidazolyl)methyl]-9-acridanon-(2-thiazolidinyliden)hydrazon,
10-[2-[4-[2-(Dimethylamino)acetyl]-1-piperazinyl]äthyl]-9-acridanon-(2-thiazolidinyliden)hydrazon,
1-Chlor-10-[2-(diäthylamino)äthyl]-9-acridanon-(2-thiazolidinyliden)hydrazon,
10-(2-Aminoäthyl)-9-acridanon-(2-thiazolidinyliden)hydrazon,
10-[2-[(Methylsulphonyl)amino]äthyl]-9-acridanon-(2-thiazolidinyliden)hydrazon,
10-[4-(Diäthylamino)butyl]-9-acridanon-(2-thiazolidinyliden)hydrazon,
10-[5-(Diäthylamino)pentyl]-9-acridanon-(2-thiazolidinyliden)hydrazon,
10-[2-(Diäthylamino)äthyl]-9-acridanon-methyl(2-thiazolin-2-yl)hydrazon und
10-[2-(Diäthylamino)äthyl]-9-acridanon-(3-methyl-2-thiazolidinyliden)hydrazon.

23. 10-[2-(Diäthylamino)äthyl]-9-acridanon-methyl-(2-thiazolyl)hydrazon,
10-[2-(4-Morpholinyl)äthyl]-9-acridanon-(2-thiazolyl)hydrazon,
10-[2-(4-Äthoxycarbonyl-1-piperazinyl]-äthyl-9-acridanon-(2-thiazolyl)hydrazon,
10-[2-(4-Pivaloyl-1-piperazinyl)äthyl]-9-acridanon-(2-thiazolyl)hydrazon,
10-[2-[4-(Methylsulphonyl)-1-piperazinyl]-äthyl]-9-acridanon-(2-thiazolyl)hydrazon,
10-[2-[4-[2-(Diäthylamino)äthyl]-1-piperazinyl]äthyl]-9-acridanon-(2-thiazolyl)-hydrazon und
10-[2-(4-Propyl-1-piperazinyl)äthyl]-9-acridanon-(2-thiazolyl)hydrazon.

24. Verbindungen gemäss einem der Ansprüche 1 bis 23 als pharmazeutische Wirkstoffe.

25. Verbindungen gemäss einem der Ansprüche 1 bis 23 als schistosomizide Stoffe.

26. Verfahren zur Herstellung von Verbindungen gemäss einem der Ansprüche 1 bis 23, dadurch gekennzeichnet, dass man

a) eine Verbindung der allgemeinen Formel

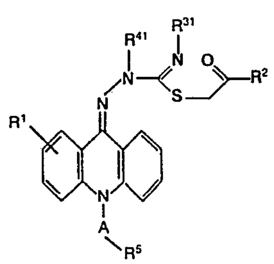
II,
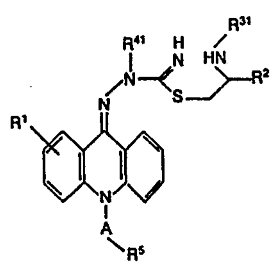
III,
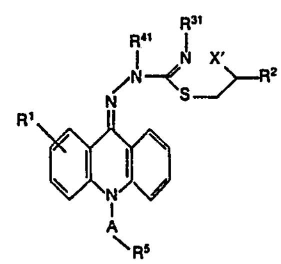
IV,
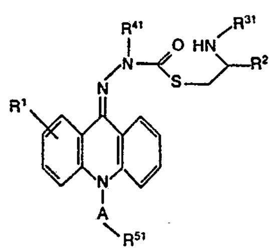
V,
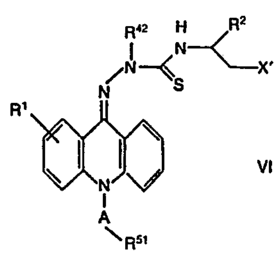
VI oder
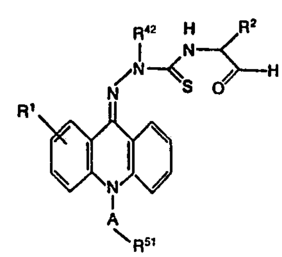
VII

worin eines der Symbole $R^{31}$ und $R^{41}$ Wasserstoff oder niederes Alkyl und das andere Wasserstoff, $R^{42}$ Wasserstoff oder niederes Alkyl, $R^{51}$ einen Rest $R^5$ gemäss Anspruch 1, der jedoch keine primäre oder sekundäre, basische Aminogruppe enthält, und X′ eine Abgangsgruppe bedeuten, und $R^1$, $R^2$ und $R^5$ die in Anspruch 1 angegebene Bedeutung besitzen,
cyclisiert, oder

b) eine Verbindung der allgemeinen Formel

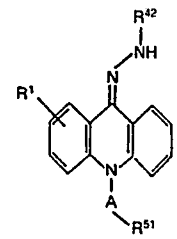
VIII

worin A und $R^1$ die in Anspruch 1 angegebene und $R^{42}$ und $R^{51}$ obige Bedeutung besitzen, mit einer Verbindung der allgemeinen Formel

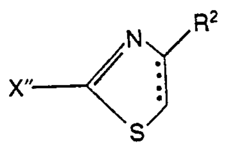

IX

worin X'' eine Abgangsgruppe bedeutet, und die punktierte Linie und $R^2$ die in Anspruch 1 angegebene Bedeutung besitzen,
umsetzt, oder

c) eine Verbindung der allgemeinen Formel

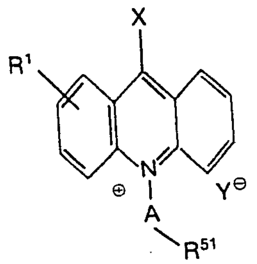

X

worin $Y^\ominus$ ein Anion und X eine Abgangsgruppe bedeuten, und A und $R^1$ die in Anspruch 1 angegebene und $R^{51}$ obige Bedeutung besitzen,
mit einer Verbindung der allgemeinen Formel

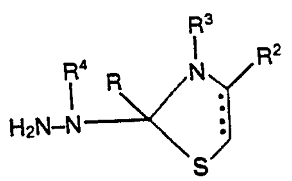

XI

worin die punktierte Linie, R, $R^2$, $R^3$ und $R^4$ die in Anspruch 1 angegebene Bedeutung besitzen,
umsetzt oder

d) die N-Schutzgruppe in einer Verbindung der allgemeinen Formel

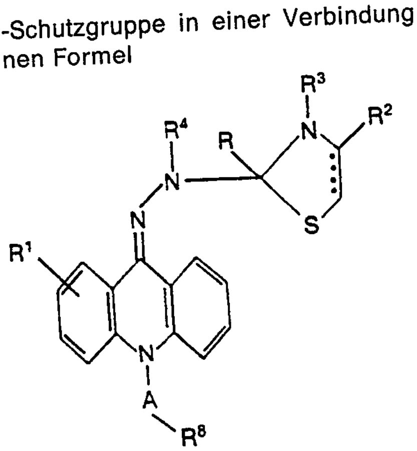

XII

worin $R^8$ einen Rest $R^5$ gemäss Anspruch 1 bedeutet, welcher eine durch eine N-Schutzgruppe blockierte primäre oder sekundäre, basische Aminogruppe enthält, und die punktierte Linie, R, $R^1$, $R^2$, $R^3$ und $R^4$ die in Anspruch 1 angegebene Bedeutung besitzen,
abspaltet, und

e) erwünschtenfalls eine erhaltene Verbindung der Formel I in ein pharmazeutisch annehmbares Säureadditionssalz überführt.

27. Arzneimittel, enthaltend eine Verbindung gemäss einem der Ansprüche 1 bis 23.

28. Schistosomizide Mittel, enthaltend eine Verbindung gemäss einem der Ansprüche 1 bis 23.

29. Verwendung von Verbindungen gemäss einem der Ansprüche 1 bis 23 zur Herstellung von Mitteln gemäss Anspruch 27 oder 28.

**Patentansprüche für den Vertragsstaat AT**

1. Verfahren zur Herstellung von Acridanon-Derivaten der allgemeinen Formel

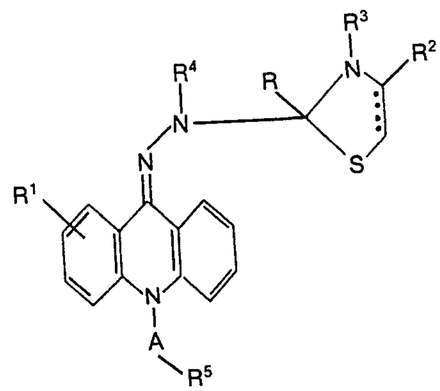

I

worin die punktierte Linie eine fakultative Bindung, $R^1$ Wasserstoff, Halogen oder Nitro, $R^2$ Wasserstoff oder niederes Alkyl, eines der Symbole $R^3$ und $R^4$ Wasserstoff oder niederes Alkyl und das andere zusammen mit R eine zusätzliche Bindung, A niederes Alkylen, $R^5$ einen 5-gliedrigen, stickstoffhaltigen, aromatischen, gegebenenfalls durch niederes Alkyl substituierten Heterocyclus, Amino oder eine Gruppe

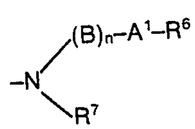 oder 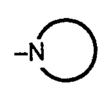 ,

das Symbol 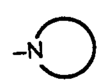

einen 5- oder 6-gliedrigen, gegebenenfalls durch niederes Alkyl substituierten gesättigten Heterocyclus, der als Ringglied noch ein Sauerstoff- oder Schwefelatom oder die Gruppe >NH oder $>N(B)_n-A^1-R^6$ enthalten kann, B die Gruppe –CO–, –COO– oder $-SO_2-$, n die Zahl 0 oder 1, $A^1$ niederes Alkylen, $R^6$ Wasserstoff, Amino, niederes Alkylamino oder Di(niederes Alkyl)amino und $R^7$ Wasserstoff oder niederes Alkyl bedeuten, und die als nieder bezeichneten Reste 1 bis 7 Kohlenstoffatome enthalten, und pharmazeutisch annehmbare Säureadditionssalze davon,
dadurch gekennzeichnet, dass man

a) eine Verbindung der allgemeinen Formel

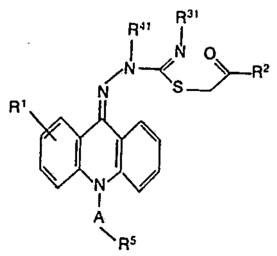

II,

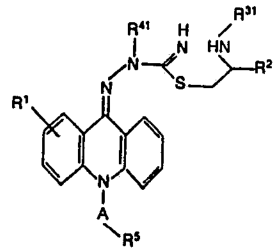

III,

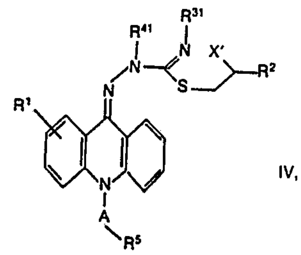

IV,

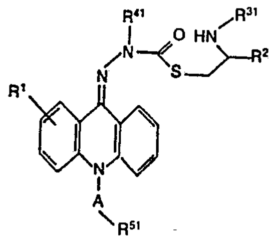

V,

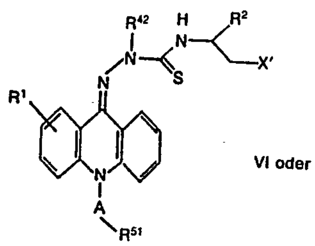

VI oder

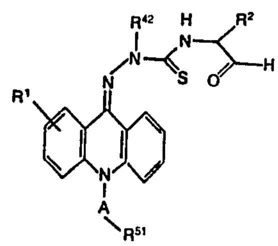

VII

worin eines der Symbole $R^{31}$ und $R^{41}$ Wasserstoff oder niederes Alkyl und das andere Wasserstoff, $R^{42}$ Wasserstoff oder niederes Alkyl, $R^{51}$ einen Rest $R^5$, der jedoch keine primäre oder sekundäre, basische Aminogruppe enthält, und X′ eine Abgangsgruppe bedeuten, und $R^1$, $R^2$ und $R^5$ obige Bedeutung besitzen,
cyclisiert, oder

b) eine Verbindung der allgemeinen Formel

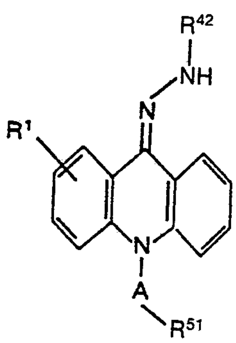

VIII

worin A, $R^1$, $R^{42}$ und $R^{51}$ obige Bedeutung besitzen, mit einer Verbindung der allgemeinen Formel

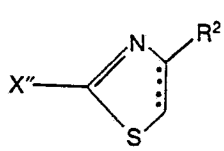

IX

worin X″ eine Abgangsgruppe bedeutet, und die punktierte Linie und $R^2$ obige Bedeutung besitzen, umsetzt, oder

c) eine Verbindung der allgemeinen Formel

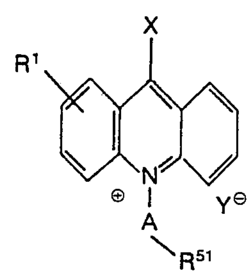

X

worin $Y^{\ominus}$ ein Anion und X eine Abgangsgruppe bedeuten, und A, $R^1$ und $R^{51}$ obige Bedeutung besitzen,
mit einer Verbindung der allgemeinen Formel

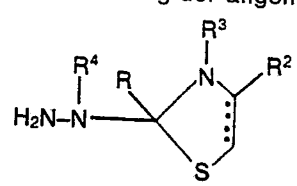

XI

worin die punktierte Linie, R, $R^2$, $R^3$ und $R^4$ obige Bedeutung besitzen,
umsetzt, oder

d) die N-Schutzgruppe in einer Verbindung der allgemeinen Formel

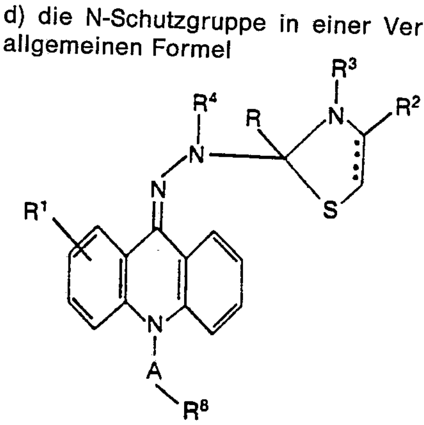

XII

worin $R^8$ einen Rest $R^5$ bedeutet, welcher eine durch eine N-Schutzgruppe blockierte primäre oder sekundäre, basische Aminogruppe enthält, und die punktierte Linie, R, $R^1$, $R^2$, $R^3$ und $R^4$ obige Bedeutung besitzen,
abspaltet, und

e) erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass eine Verbindung der Formel I hergestellt wird, worin ein allfälliger Substituent $R^1$ sich in der 1-Stellung befindet.

3. Verfahren gemäss Anspruch 1 oder 2, dadurch gekennzeichnet, dass eine Verbindung der Formel I hergestellt wird, worin $R^1$ Wasserstoff bedeutet.

4. Verfahren gemäss einem der Ansprüche 1 bis 3, dadurch gekennzeichnet, dass eine Verbindung der Formel I hergestellt wird, worin $R^2$ Wasserstoff bedeutet.

5. Verfahren gemäss einem der Ansprüche 1 bis 4, dadurch gekennzeichnet, dass eine Verbindung der Formel I hergestellt wird, worin eines der Symbole $R^3$ und $R^4$ Wasserstoff und das andere zusammen mit R eine zusätzliche Bindung bedeuten.

6. Verfahren gemäss einem der Ansprüche 1 bis 5, dadurch gekennzeichnet, dass eine Verbindung der Formel I hergestellt wird, worin A Dimethylen oder Trimethylen bedeutet.

7. Verfahren gemäss einem der Ansprüche 1 bis 6, dadurch gekennzeichnet, dass eine Verbindung der Formel I hergestellt wird, worin $R^5$ Amino, 1-Pyrrolidinyl, 1-Piperidinyl, 1-Morpholinyl, 1-Piperazinyl oder die Gruppe $-NR^7-A^1-R^{61}$ oder

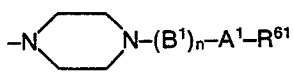 ,

$A^1$ niederes Alkylen, $B^1$ die Gruppe –CO–, n die Zahl 0 oder 1, $R^{61}$ Wasserstoff und $R^7$ Wasserstoff oder niederes Alkyl bedeuten.

8. Verfahren gemäss Anspruch 7, dadurch gekennzeichnet, dass eine Verbindung der Formel I hergestellt wird, worin $R^5$ Amino, Dimethylamino, Diäthylamino, 1-Pyrrolidinyl, 1-Piperidinyl, 4-Methyl-1-piperazinyl oder 4-Acetyl-1-piperazinyl bedeutet.

9. Verfahren gemäss Anspruch 1, dadurch gekennzeichnet, dass
10-[2-(4-(Methyl-1-piperazinyl)äthyl]-9-acridanon-(2-thiazolyl)hydrazon
hergestellt wird.

10. Verfahren gemäss Anspruch 1, dadurch gekennzeichnet, dass
10-[2-(4-Methyl-1-piperazinyl)äthyl]-9-acridanon-(2-thiazolidinyliden)hydrazon
hergestellt wird.

11. Verfahren gemäss Anspruch 1, dadurch gekennzeichnet, dass
10-[2-(4-Acetyl-1-piperazinyl)äthyl]-9-acridanon-(2-thiazolidinyliden)hydrazon
hergestellt wird.

12. Verfahren gemäss Anspruch 1, dadurch gekennzeichnet, dass
10-[2-(Dimethylamino)äthyl]-9-acridanon-(2-thiazolidinyliden)hydrazon
hergestellt wird.

13. Verfahren gemäss Anspruch 1, dadurch gekennzeichnet, dass
10-[2-(Diäthylamino)äthyl]-9-acridanon-(2-thiazolyl)hydrazon
hergestellt wird.

14. Verfahren gemäss Anspruch 1, dadurch gekennzeichnet, dass
10-[2-(1-Piperidinyl)äthyl]-9-acridanon-(2-thiazolyl)hydrazon
hergestellt wird.

15. Verfahren gemäss Anspruch 1, dadurch gekennzeichnet, dass
10-(2-Aminoäthyl)-9-acridanon-(2-thiazolyl)-hydrazon
hergestellt wird.

16. Verfahren gemäss Anspruch 1, dadurch gekennzeichnet, dass
10-[2-(Dimethylamino)äthyl]-9-acridanon-(2-thiazolyl)hydrazon
hergestellt wird.

17. Verfahren gemäss Anspruch 1, dadurch gekennzeichnet, dass
10-[2-(4-Acetyl-1-piperazinyl)äthyl]-9-acridanon-(2-thiazolyl)hydrazon
hergestellt wird.

18. Verfahren gemäss Anspruch 1, dadurch gekennzeichnet, dass
10-[3-(Dimethylamino)propyl]-9-acridanon-(2-thiazolyl)hydrazon
hergestellt wird.

19. Verfahren gemäss Anspruch 1, dadurch gekennzeichnet, dass
10-[2-(Diäthylamino)äthyl]-9-acridanon-(2-thiazolidinyliden)hydrazon
hergestellt wird.

20. Verfahren gemäss Anspruch 1, dadurch gekennzeichnet, dass
10-[2-(1-Pyrrolidinyl)äthyl]-9-acridanon-(2-thiazolidinyliden)hydrazon
hergestellt wird.

21. Verfahren gemäss Anspruch 1, dadurch gekennzeichnet, dass
10-[3-(Dimethylamino)propyl]-9-acridanon-(2-thiazolidinyliden)hydrazon
hergestellt wird.

22. Verfahren gemäss Anspruch 1, dadurch gekennzeichnet, dass
10-[2-(4-Propyl-1-piperazinyl)äthyl]-9-acridanon-(2-thiazolidinyliden)hydrazon,
10-[2-(Dimethylamino)propyl]-9-acridanon-(2-thiazolidinyliden)hydrazon,
10-[2-(1-Piperidinyl)äthyl]-9-acridanon-(2-thiazolidinyliden)hydrazon,
10-[2-(2,6-Dimethyl-1-piperidinyl)äthyl]-9-acridanon-(2-thiazolidinyliden)hydrazon,
10-[2-(4-Morpholinyl)äthyl]-9-acridanon-(2-thiazolidinyliden)hydrazon,
10-[2-(4-Äthoxycarbonyl-1-piperazinyl)-äthyl]-9-acridanon-(2-thiazolidinyliden)-hydrazon,

10-[2-(4-Pivaloyl-1-piperazinyl)äthyl]-9-acridanon-(2-thiazolidinyliden)hydrazon,
10-[2-[4-(Methylsulfonyl)-1-piperazinyl]-äthyl]-9-acridanon-(2-thiazolidinyliden)-hydrazon,
10-[(1-Methyl-4-imidazolyl)methyl]-9-acridanon-(2-thiazolidinyliden)hydrazon,
10-[2-[4-[2-(Dimethylamino)acetyl]-1-piperazinyl]äthyl]-9-acridanon-(2-thiazolidinyliden)hydrazon,
1-Chlor-10-[2-(diäthylamino)äthyl]-9-acridanon-(2-thiazolidinyliden)hydrazon,
10-(2-Aminoäthyl)-9-acridanon-(2-thiazolidinyliden)hydrazon,
10-[2-[(Methylsulphonyl)amino]äthyl]-9-acridanon-(2-thiazolidinyliden)hydrazon,
10-[4-(Diäthylamino)butyl]-9-acridanon-(2-thiazolidinyliden)hydrazon,
10-[5-(Diäthylamino)pentyl]-9-acridanon-(2-thiazolidinyliden)hydrazon,
10-[2-(Diäthylamino)äthyl]-9-acridanon-methyl(2-thiazolin-2-yl)hydrazon und
10-[2-(Diäthylamino)äthyl-9-acridanon-(3-methyl-2-thiazolidinyliden)hydrazon
hergestellt wird.

23. Verfahren gemäss Anspruch 1, dadurch gekennzeichnet, dass
10-[2-(Diäthylamino)äthyl]-9-acridanon-methyl-(2-thiazolyl)hydrazon,
10-[2-(4-Morpholinyl)äthyl]-9-acridanon-(2-thiazolyl)hydrazon,
10-[2-(4-Äthoxycarbonyl-1-piperazinyl]-äthyl-9-acridanon-(2-thiazolyl)hydrazon,
10-[2-(4-Pivaloyl-1-piperazinyl)äthyl]-9-acridanon-(2-thiazolyl)hydrazon,
10-[2-[4-(Methylsulphonyl)-1-piperazinyl]-äthyl]-9-acridanon-(2-thiazolyl)hydrazon,
10-[2-[4-[2-(Diäthylamino)äthyl]-1-piperazinyl]-äthyl]-9-acridanon-(2-thiazolyl)hydrazon
und
10-[2-(4-Propyl-1-piperazinyl)äthyl]-9-acridanon-(2-thiazolyl)hydrazon
hergestellt wird.

24. Verwendung von Verbindungen der in Anspruch 1 definierten Formel I zur Herstellung von Arzneimitteln.

25. Verwendung von Verbindungen der in Anspruch 1 definierten Formel I zur Herstellung von schistosomiziden Mitteln.

**Claims for the Contracting States BE CH DE FR GB IT LI LU NL SE**

1. Acridanone derivatives of the general formula

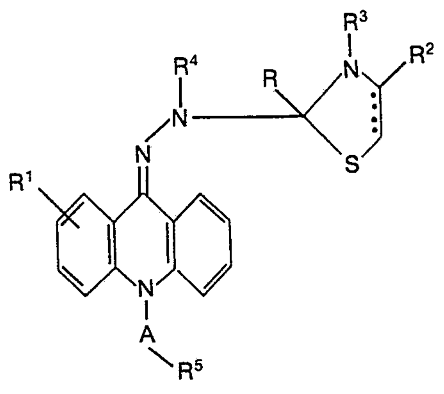

I

wherein the dotted line signifies an optional bond, $R^1$ signifies hydrogen, halogen or nitro, $R^2$ signifies hydrogen or lower alkyl, one of the symbols $R^3$ and $R^4$ signifies hydrogen or lower alkyl and the other together with R signifies an additional bond, A signifies lower alkylene, $R^5$ signifies a 5-membered, nitrogen-containing, optionally lower alkylsubstituted aromatic heterocycle, amino or a group

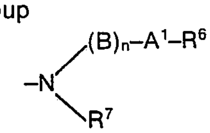 or 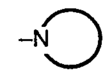 ,

the symbol 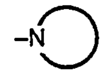

signifies a 5- or 6-membered, optionally lower-alkyl substituted saturated heterocycle which can contain as a ring member an oxygen or sulphur atom or the group >NH or $>N(B)_n-A^1-R^6$, B signifies the group –CO–, –COO– or $-SO_2-$, n signifies the number 0 or 1, $A^1$ signifies lower alkylene, $R^6$ signifies hydrogen, amino, lower alkylamino or di(lower alkyl)amino and $R^7$ signifies hydrogen or lower alkyl, and the residues denoted as lower contain 1 to 7 carbon atoms,
and pharmaceutically acceptable acid addition salts thereof.

2. Compounds in accordance with claim 1, wherein a substituent $R^1$ which may be present is situated in the 1-position.

3. Compounds in accordance with claim 1 or 2, wherein $R^1$ signifies hydrogen.

4. Compounds in accordance with any one of claims 1 to 3, wherein $R^2$ signifies hydrogen.

5. Compounds in accordance with any one of claims 1 to 4, wherein one of the symbols $R^3$ and $R^4$ signifies hydrogen and the other together with R signifies an additional bond.

6. Compounds in accordance with any one of claims 1 to 5, wherein A signifies dimethylene or trimethylene.

7. Compounds in accordance with any one of claims 1 to 6, wherein $R^5$ signifies amino, 1-pyrrolidinyl, 1-piperidinyl, 1-morpholinyl, 1-piperazinyl or the group $-NR^7-A^1-R^{61}$ or

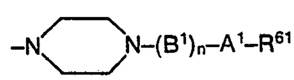 ,

$A^1$ signifies lower alkylene, $B^1$ signifies the group –CO–, n signifies the number 0 or 1, $R^{61}$ signifies hydrogen and $R^7$ signifies hydrogen or lower alkyl.

8. Compounds in accordance with claim 7, wherein $R^5$ signifies amino, dimethylamino, diethylamino, 1-pyrrolidinyl, 1-piperidinyl, 4-methyl-1-piperazinyl or 4-acetyl-1-piperazinyl.

9. 10-[2-(4-(Methyl-1-piperazinyl)-ethyl]-9-acridanone (2-thiazolyl)hydrazone.

10. 10-[2-(4-Methyl-1-piperazinyl)-ethyl]-9-acridanone (2-thiazolidinylidene)-hydrazone.

11. 10-[2-(4-Acetyl-1-piperazinyl)-ethyl]-9-acridanone (2-thiazolidinylidene)-hydrazone.

12. 10-[2-(Dimethylamino)ethyl]-9-acridanone (2-thiazolidinylidene)hydrazone.

13. 10-[2-(Diethylamino)ethyl]-9-acridanone (2-thiazolyl)hydrazone.

14. 10-[2-(1-Piperidinyl)ethyl]-9-acridanone (2-thiazolyl)hydrazone.

15. 10-(2-Aminoethyl)-9-acridanone (2-thiazolyl)hydrazone.

16. 10-[2-(Dimethylamino)ethyl]-9-acridanone (2-thiazolyl)hydrazone.

17. 10-[2-(4-Acetyl-1-piperazinyl)-ethyl]-9-acridanone (2-thiazolyl)hydrazone.

18. 10-[3-(Dimethylamino)propyl]-9-acridanone (2-thiazolyl)hydrazone.

19. 10-[2-(Diethylamino)ethyl]-9-acridanone (2-thiazolidinylidene)hydrazone.

20. 10-[2-(1-Pyrrolidinyl)ethyl]-9-acridanone (2-thiazolidinylidene)hydrazone.

21. 10-[3-(Dimethylamino)propyl]-9-acridanone (2-thiazolidinylidene)hydrazone.

22. 10-[2-(4-Propyl-1-piperazinyl)-ethyl]-9-acridanone (2-thiazolidinylidene)-hydrazone,
10-[2-(dimethylamino)propyl]-9-acridanone (2-thiazolidinylidene)hydrazone,
10-[2-(1-piperidinyl)ethyl]-9-acridanone (2-thiazolidinylidene)hydrazone,
10-[2-(2,6-dimethyl-1-piperidinyl)-ethyl]-9-acridanone (2-thiazolidinylidene)-hydrazone,
10-[2-(4-morpholinyl)ethyl]-9-acridanone (2-thiazolidinylidene)hydrazone,
10-[2-(4-ethoxycarbonyl-1-piperazinyl)-ethyl]-9-acridanone (2-thiazolidinylidene)-hydrazone,
10-[2-(4-pivaloyl-1-piperazinyl)ethyl]-9-acridanone (2-thiazolidinylidene)hydrazone,
10-[2-[4-(methylsulphonyl)-1-piperazinyl)-ethyl]-9-acridanone (2-thiazolidinylidene)-hydrazone,
10-[(1-methyl-4-imidazolyl)methyl]-9-acridanone (2-thiazolidinylidene)hydrazone,
10-[2-[4-[2-(dimethylamino)acetyl]-1-piperazinyl]ethyl]-9-acridanone (2-thiazolidinylidene)hydrazone,
1-chloro-10-[2-(diethylamino)ethyl]-9-acridanone (2-thiazolidinylidene)hydrazone,
10-(2-aminoethyl)-9-acridanone (2-thiazolidinylidene)hydrazone,
10-[2-[(methylsulphonyl)amino]ethyl]-9-acridanone (2-thiazolidinylidene)hydrazone,
10-[4-(diethylamino)butyl]-9-acridanone (2-thiazolidinylidene)hydrazone,
10-[5-(diethylamino)pentyl]-9-acridanone (2-thiazolidinylidene)hydrazone,
10-[2-(diethylamino)ethyl]-9-acridanone methyl(2-thiazolin-2-yl)hydrazone and
10-[2-(diethylamino)ethyl]-9-acridanone (3-methyl-2-thiazolidinylidene)hydrazone.

23. 10-[2-Diethylamino)ethyl]-9-acridanone methyl-(2-thiazolyl)hydrazone,
10-[2-(4-morpholinyl)ethyl]-9-acridanone (2-thiazolyl)hydrazone,
10-[2-(4-ethoxycarbonyl-1-piperazinyl)-ethyl-9-acridanone (2-thiazolyl)hydrazone,
10-[2-(4-pivaloyl-1-piperazinyl)ethyl]-9-acridanone (2-thiazolyl)hydrazone,
10-[2-[4-(methylsulphonyl)-1-piperazinyl]-ethyl]-9-acridanone (2-thiazolyl)hydrazone,
10-[2-[4-[2-(diethylamino)ethyl]-1-piperazinyl]ethyl]-9-acridanone (2-thiazolyl)hydrazone and
10-[2-(4-propyl-1-piperazinyl)ethyl]-9-acridanone (2-thiazolyl)hydrazone.

24. Compounds in accordance with any one of claims 1 to 23 as pharmaceutically active substances.

25. Compounds in accordance with any one of claims 1 to 23 as schistosomicidal substances.

26. A process for the manufacture of compounds in accordance with any one of claims 1 to 23, characterized by

a) cyclizing a compound of the general formula

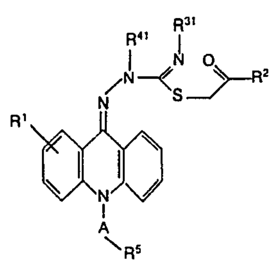
II,
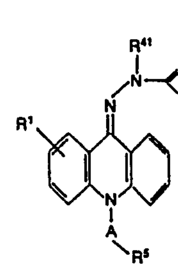
III,
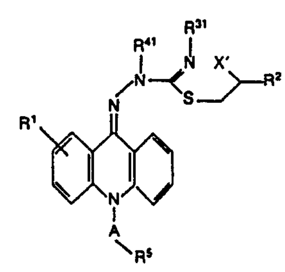
IV,
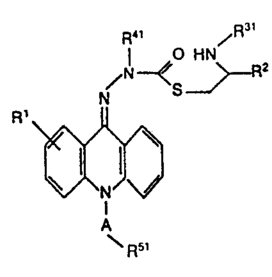
V,
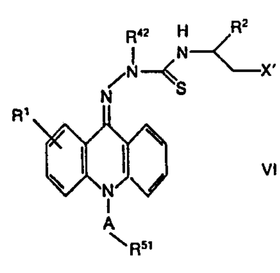
VI or
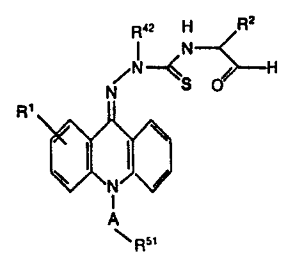
VII

wherein one of the symbols $R^{31}$ and $R^{41}$ signifies hydrogen or lower alkyl and the other signifies hydrogen, $R^{42}$ signifies hydrogen or lower alkyl, $R^{51}$ signifies a residue $R^5$ in accordance with claim 1, but does not contain a primary or secondary basic amino group, and X′ signifies a leaving group, and $R^1$, $R^2$ and $R^5$ have the significance given in claim 1,
or

b) reacting a compound of the general formula

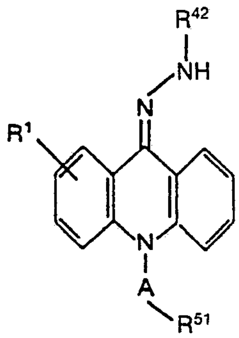

VIII

wherein A and $R^1$ have the significance given in claim 1 and $R^{42}$ and $R^{51}$ have the above significance,
with a compound of the general formula

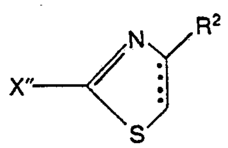

IX

wherein X″ signifies a leaving group and the dotted line and $R^2$ have the significance given in claim 1,
or

c) reacting a compound of the general formula

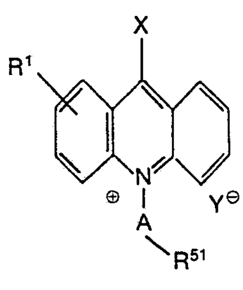

X

wherein $Y^{\ominus}$ signifies an anion and X signifies a leaving group, and A and $R^1$ have the significance given in claim 1 and $R^{51}$ has the above significance,
with a compound of the general formula

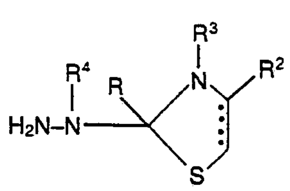

XI

wherein the dotted line, R, $R^2$, $R^3$ and $R^4$ have the significance given in claim 1,
or

d) cleaving off the N-protecting group in a compound of the general formula

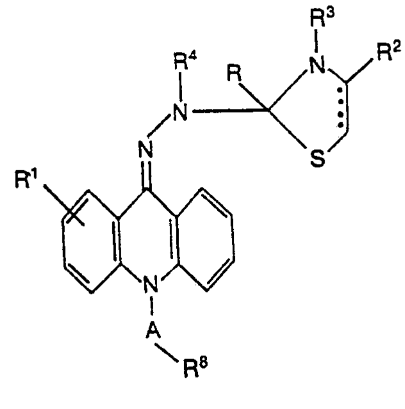

XII

wherein $R^8$ signifies a residue $R^5$ in accordance with claim 1 which contains a primary or secondary basic amino group blocked by a N-protecting group and the dotted line, R, $R^1$, $R^2$, $R^3$ and $R^4$ have the significance given in claim 1,
and

e) if desired, converting a compound of formula I obtained into a pharmaceutically acceptable acid addition salt.

27. A medicament containing a compound in accordance with any one of claims 1 to 23.

28. A schistosomicidal medicament containing a compound in accordance with any one of claims 1 to 23.

29. The use of compounds in accordance with any one of claims 1 to 23 for the manufacture of medicaments in accordance with claim 27 or 28.

**Claims for the Contracting State AT**

1. A process for the manufacture of acridanone derivatives of the general formula

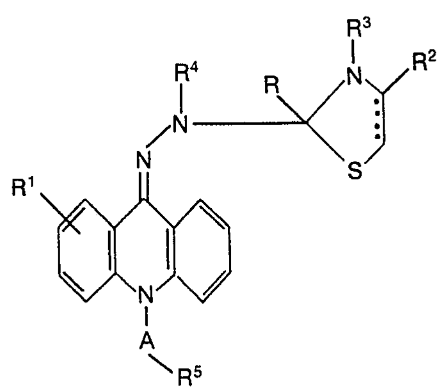

I

wherein the dotted line signifies an optional bond, $R^1$ signifies hydrogen, halogen or nitro, $R^2$ signifies hydrogen or lower alkyl, one of the symbols $R^3$ and $R^4$ signifies hydrogen or lower alkyl and the other together with R signifies an additional bond, A signifies lower alkylene, $R^5$ signifies a 5-mem-

bered, nitrogen-containing optionally lower alkylsubstituted aromatic heterocycle, amino or a group

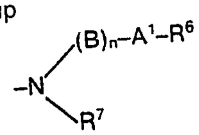
or
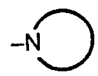
,

the symbol 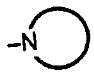

signifies a 5- or 6-membered, optionally lower-alkyl substituted saturated heterocycle which can contain as a ring member an oxygen or sulphur atom or the group >NH or $>N(B)_n-A^1-R^6$, B signifies the group –CO–, –COO– or $-SO_2-$, n signifies the number 0 or 1, $A^1$ signifies lower alkylene, $R^6$ signifies hydrogen, amino, lower alkylamino or di(lower alkyl)amino and $R^7$ signifies hydrogen or lower alkyl, and the residues denoted as lower contain 1 to 7 carbon atoms, and pharmaceutically acceptable acid addition salts thereof, characterized by

a) cyclizing a compound of the general formula

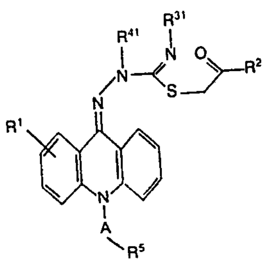
II,
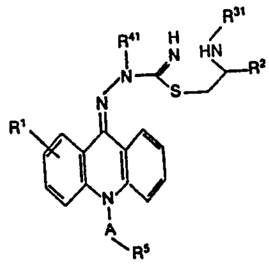
III,
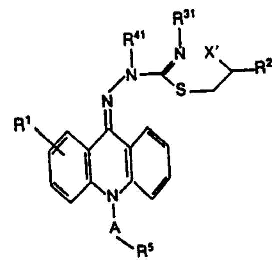
IV,

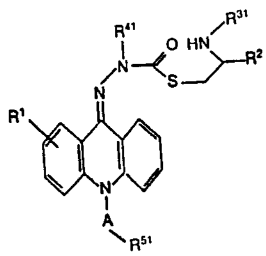
V,
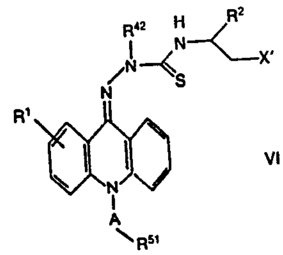
VI or
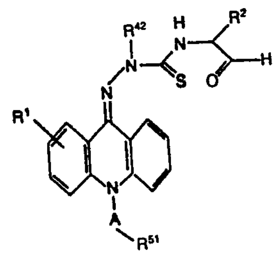
VII

wherein one of the symbols $R^{31}$ and $R^{41}$ signifies hydrogen or lower alkyl and the other signifies hydrogen, $R^{42}$ signifies hydrogen or lower alkyl, $R^{51}$ signifies a residue $R^5$, but does not contain a primary or secondary basic amino group, and X' signifies a leaving group, and $R^1$, $R^2$ and $R^5$ have the above significance,

b) reacting a compound of the general formula

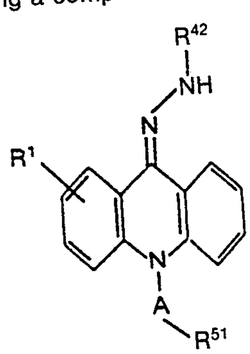
VIII

wherein A, $R^1$, $R^{42}$ and $R^{51}$ have the above significance,
with a compound of the general formula

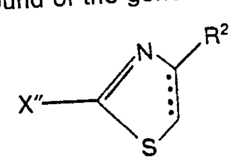
IX

wherein X'' signifies a leaving group and the dotted line and $R^2$ have the above significance,
or

c) reacting a compound of the general formula

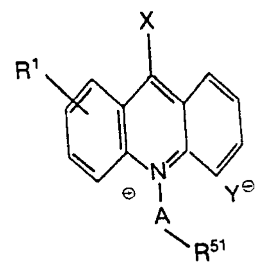
X

wherein $Y^{\ominus}$ signifies an anion and X signifies a leaving group, and A, $R^1$ and $R^{51}$ have the above significance,
with a compound of the general formula

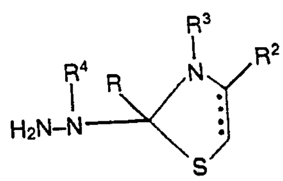
XI

wherein the dotted line, R, $R^2$, $R^3$ and $R^4$ have the above significance,
or

d) cleaving off the N-protecting group in a compound of the general formula

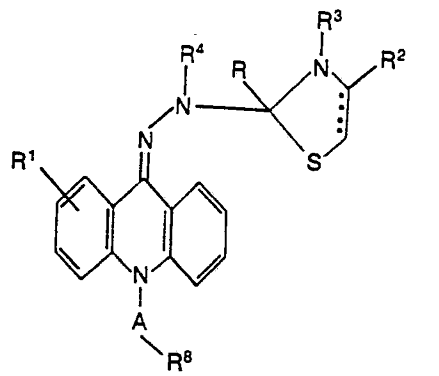

XII

wherein $R^8$ signifies a residue $R^5$ which contains a primary or secondary basic amino group blocked by a N-protecting group and the dotted line, R, $R^1$, $R^2$, $R^3$ and $R^4$ have the above significance, and

e) if desired, converting a compound of formula I obtained into a pharmaceutically acceptable acid addition salt.

2. A process in accordance with claim 1, characterized in that a compound in which a substituent $R^1$ which may be present is present in the 1-position is manufactured.

3. A process in accordance with claim 1 or 2, characterized in that a compound of formula I in which $R^1$ signifies hydrogen is manufactured.

4. A process in accordance with any one of claims 1 to 3, characterized in that a compound of formula I in which $R^2$ signifies hydrogen is manufactured.

5. A process in accordance with any one of claims 1 to 4, characterized in that a compound of formula I in which one of the symbols $R^3$ and $R^4$ signifies hydrogen and the other together with R signifies an additional bond is manufactured.

6. A process in accordance with any one of claims 1 to 5, characterized in that a compound of formula I in which A signifies dimethylene or trimethylene is manufactured.

7. A process in accordance with any one of claims 1 to 6, characterized in that a compound of formula I in which $R^5$ signifies amino, 1-pyrrolidinyl, 1-piperidinyl, 1-morpholinyl, 1-piperazinyl or the group $-NR^7-A^1-R^{61}$ or

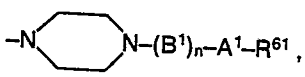 ,

$A^1$ signifies lower alkylene, $B^1$ signifies the group –CO–, n signifies the number 0 or 1, $R^{61}$ signifies hydrogen and $R^7$ signifies hydrogen or lower alkyl is manufactured.

8. A process in accordance with claim 7, characterized in that a compound of formula I in which $R^5$ signifies amino, dimethylamino, diethylamino, 1-pyrrolidinyl, 1-piperidinyl, 4-methyl-1-piperazinyl or 4-acetyl-1-piperazinyl is manufactured.

9. A process in accordance with claim 1, characterized in that 10-[2-(4-(methyl-1-piperazinyl)ethyl]-9-acridanone (2-thiazolyl)hydrazone is manufactured.

10. A process in accordance with claim 1, characterized in that 10-[2-(4-methyl-1-piperazinyl)ethyl]-9-acridanone (2-thiazolidinylidene)hydrazone is manufactured.

11. A process in accordance with claim 1, characterized in that 10-[2-(4-acetyl-1-piperazinyl)ethyl]-9-acridanone (2-thiazolidinylidene)hydrazone is manufactured.

12. A process in accordance with claim 1, characterized in that 10-[2-(dimethylamino)ethyl]-9-acridanone (2-thiazolidinylidene)hydrazone is manufactured.

13. A process in accordance with claim 1, characterized in that 10-[2-(diethylamino)ethyl]-9-acridanone (2-thiazolyl)hydrazone is manufactured.

14. A process in accordance with claim 1, characterized in that 10-[2-(1-piperidinyl)ethyl]-9-acridanone (2-thiazolyl)hydrazone is manufactured.

15. A process in accordance with claim 1, characterized in that 10-(2-aminoethyl)-9-acridanone (2-thiazolyl)hydrazone is manufactured.

16. A process in accordance with claim 1, characterized in that 10-[2-(dimethylamino)ethyl]-9-acridanone (2-thiazolyl)hydrazone is manufactured.

17. A process in accordance with claim 1, characterized in that 10-[2-(4-acetyl-1-piperazinyl)ethyl]-9-acridanone (2-thiazolyl-)hydrazone is manufactured.

18. A process in accordance with claim 1, characterized in that 10-[3-(dimethylamino)propyl]-9-acridanone (2-thiazolyl)hydrazone is manufactured.

19. A process in accordance with claim 1, characterized in that 10-[2-(diethylamino)ethyl]-9-acridanone (2-thiazolidinylidene)hydrazone is manufactured.

20. A process in accordance with claim 1, characterized in that 10-[2-(1-pyrrolidinyl)ethyl]-9-acridanone (2-thiazolidinylidene)hydrazone is manufactured.

21. A process in accordance with claim 1, characterized in that 10-[3-(dimethylamino)propyl]-9-acridanone (2-thiazolidinylidene)hydrazone is manufactured.

22. A process in accordance with claim 1, characterized in that
10-[2-(4-propyl-1-piperazinyl)ethyl]-9-acridanone (2-thiazolidinylidene)hydrazone,
10-[2-(dimethylamino)propyl]-9-acridanone (2-thiazolidinylidene)hydrazone,
10-[2-(1-piperidinyl)ethyl]-9-acridanone (2-thiazolidinylidene)hydrazone,
10-[2-(2,6-dimethyl-1-piperidinyl)-ethyl]-9-acridanone (2-thiazolidinylidene)-hydrazone,
10-[2-(4-morpholinyl)ethyl]-9-acridanone (2-thiazolidinylidene)hydrazone,
10-[2-(4-ethoxycarbonyl-1-piperazinyl)-ethyl]-9-acridanone (2-thiazolidinylidene)hydrazone,
10-[2-(4-pivaloyl-1-piperazinyl)-ethyl]-9-acridanone (2-thiazolidinylidene)-hydrazone,
10-[2-[4-(methylsulphonyl)-1-piperazinyl]-ethyl]-9-acridanone (2-thiazolidinylidene)hydrazone,
10-[(1-methyl-4-imidazolyl)methyl]-9-acridanone (2-thiazolidinylidene)hydrazone,
10-[2-[4-[2-(dimethylamino)acetyl]-1-piperazinyl]ethyl]-9-acridanone (2-thiazolidinylidene)hydrazone,
1-chloro-10-[2-(diethylamino)ethyl]-9-acridanone (2-thiazolidinylidene)-hydrazone,
10-(2-aminoethyl)-9-acridanone (2-thiazolidinylidene)hydrazone,
10-[2-[(methylsulphonyl)amino]ethyl]-9-acridanone (2-thiazolidinylidene)-hydrazone,
10-[4-(diethylamino)butyl]-9-acridanone-(2-thiazolidinylidene)hydrazone,
10-[5-(diethylamino)pentyl]-9-acridanone (2-thiazolidinylidene)hydrazone,
10-[2-(diethylamino)ethyl]-9-acridanone methyl(2-thiazolin-2-yl)hydrazone and
10-[2-(diethylamino)ethyl]-9-acridanone (3-methyl-2-thiazolidinylidene)hydrazone
is manufactured.

23. A process in accordance with claim 1, characterized in that
10-[2-(diethylamino)ethyl]-9-acridanone methyl-(2-thiazolyl)hydrazone,
10-[2-(4-morpholinyl)ethyl]-9-acridanone (2-thiazolyl)hydrazone,
10-[2-(4-ethoxycarbonyl-1-piperazinyl)-ethyl]-9-acridanone (2-thiazolyl)hydrazone,
10-[2-(4-pivaloyl-1-piperazinyl)ethyl]-9-acridanone (2-thiazolyl)hydrazone,
10-[2-[4-(methylsulphonyl)-1-piperazinyl]-ethyl]-9-acridanone (2-thiazolyl)hydrazone,
10-[2-[4-[2-(diethylamino)ethyl]-1-piperazinyl]ethyl]-9-acridanone (2-thiazolyl)hydrazone and
10-[2-(4-propyl-1-piperazinyl)ethyl]-9-acridanone (2-thiazolyl)hydrazone
is manufactured.

24. The use of compounds of formula I defined in claim 1 for the manufacture of medicaments.

25. The use of compounds of formula I defined in claim 1 for the manufacture of schistosomicidal medicaments.

**Revendications pour les Etats Contractants BE, CH, DE, FR, GB, IT, LI, LU, NL, SE**

1. Dérivés de l'acridanone répondant à la formule générale

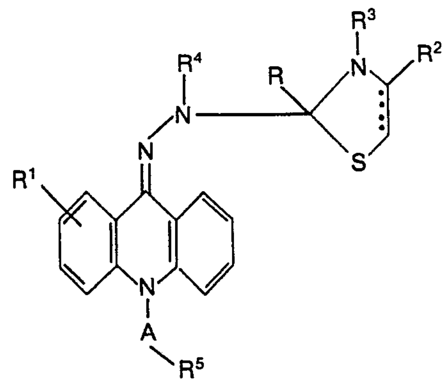

I

dans laquelle le trait en pointillé représente une liaison facultative, $R^1$ représente l'hydrogène, un halogène ou un groupe nitro, $R^2$ représente l'hydrogène ou un groupe alkyle inférieur, l'un des symboles $R^3$ et $R^4$ représente l'hydrogène ou un groupe alkyle inférieur, et l'autre forme avec R une liaison supplèmentaire, A représente un groupe alkylène inférieur, $R^5$ représente un hétérocycle aromatique azoté à 5 chaînons éventuellement substitué par un groupe alkyle inférieur, un groupe amino ou un groupe

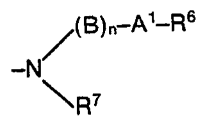

ou

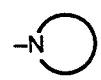

le symbole 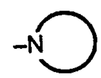

représente un hétérocycle saturé à 5 ou 6 chaînons, éventuellement substitué par un groupe alkyle inférieur et qui peut encore contenir en tant que chaînon cyclique un atome d'oxygène ou de soufre ou le groupe >NH ou $>N(B)_n$–$A^1$–$R^6$, B représente un groupe –CO–, –COO– ou $–SO_2–$, n est égal à 0 ou 1, $A^1$ représente un groupe alkylène inférieur, $R^6$ représente l'hydrogène, un groupe amino, alkylamino inférieur ou di-(alkyle inférieur)-amino et $R^7$ représente l'hydrogène ou un groupe alkyle inférieur, les groupes qualifiés d'«inférieurs» contenant de 1 à 7 atomes de carbone,
et leurs sels acceptables pour l'usage pharmaceutique formés par addition avec des acides.

2. Composés selon la revendication 1, dans lesquels un substituant éventuel $R^1$ se trouve en position 1.

3. Composés selon la revendication 1 ou 2, dans lesquels $R^1$ représente l'hydrogène.

4. Composés selon l'une des revendications 1 à 3, dans lesquels $R^2$ représente l'hydrogène.

5. Composés selon l'une des revendications 1 à 4, dans lesquels l'un des symboles $R^3$ et $R^4$ représente l'hydrogène et l'autre forme avec R une liaison supplémentaire.

6. Composés selon l'une des revendications 1 à 5, dans lesquels A représente un groupe diméthylène ou triméthylène.

7. Composés selon l'une des revendications 1 à 6, dans lesquels $R^5$ représente un groupe amino, 1-pyrrolidinyle, 1-pipéridinyle, 1-morpholinyle, 1-pipérazinyle ou le groupe $-NR^7-A^1-R^{61}$ ou

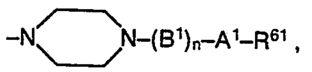

$A^1$ représente un groupe alkylène inférieur, $B^1$ représente le groupe –CO–, n est égal à 0 ou 1, $R^{61}$ représente l'hydrogène et $R^7$ l'hydrogène ou un groupe alkyle inférieur.

8. Composés selon la revendication 7, dans lesquels $R^5$ représente un groupe amino, diméthylamino, diéthylamino, 1-pyrrolidinyle, 1-pipéridinyle, 4-méthyl-1-pipérazinyle ou 4-acétyl-1-pipérazinyle.

9. La 10-[2-(4-méthyl-1-pipérazinyl)-éthyl]-9-acridanone-(2-thiazolyl)-hydrazone.

10. La 10-[2-(4-méthyl-1-pipérazinyl)-éthyl]-9-acridanone-(2-thiazolidinylidène)-hydrazone.

11. La 10-[2-(4-acéthyl-1-pipérazinyl)-éthyl]-9-acridanone-(2-thiazolidinylidène)-hydrazone.

12. La 10-[2-(diméthylamino)éthyl]-9-acridanone-(2-thiazolidinylidène)-hydrazone.

13. La 10-[2-(diéthylamino)éthyl]-9-acridanone-(2-thiazolyl)-hydrazone.

14. La 10-[2-(1-pipéridinyl)éthyl]-9-acridanone-(2-thiazolyl)-hydrazone.

15. La 10-(2-aminoéthyl)-9-acridanone-(2-thiazolyl)-hydrazone.

16. La 10-[2-(diméthylamino)éthyl]-9-acridanone-(2-thiazolyl)-hydrazone.

17. La 10-[2-(4-acétyl-1-pipérazinyl)éthyl]-9-acridanone-(2-thiazolyl)-hydrazone.

18. La 10-[3-(diméthylamino)propyl]-9-acridanone-(2-thiazolyl)-hydrazone.

19. La 10-[2-(diéthylamino)éthyl]-9-acridanone (2-thiazolidinylidène)-hydrazone.

20. La 10-[2-(1-pyrrolidinyl)éthyl]-9-acridanone-(2-thiazolidinylidène)-hydrazone.

21. La 10-[3-(diméthylamino)propyl]-9-acridanone-(2-thiazolidinylidène)-hydrazone.

22. La 10-[2-(4-propyl-1-pipérazinyl)éthyl]-9-acridanone-(2-thiazolidinylidène)-hydrazone, la 10-[2-(diméthylamino)propyl]-9-acridanone-(2-thiazolidinylidène)-hydrazone, la 10-[2-(1-pipéridinyl)éthyl]-9-acridanone-(2-thiazolidinylidène)-hydrazone, la 10-[2-(2,6-diméthyl-1-pipéridinyl)éthyl]-9-acridanone-(2-thiazolidinylidène)-hydrazone, la 10-[2-(4-morpholinyl)éthyl]-9-acridanone-(2-thiazolidinylidène)-hydrazone, la 10-[2-(4-éthoxycarbonyl-1-pipérazinyl)éthyl]-9-acridanone-(2-thiazolidinylidène)-hydrazone, la 10-[2-(4-pivaloyl-1-pipérazinyl)éthyl]-9-acridanone-(2-thiazolidinylidène)-hydrazone, la 10-[2-[4-(méthylsulfonyl)-1-pipérazinyl]-éthyl]-9-acridanone-(2-thiazolidinylidène)-hydrazone, la 10-[(1-méthyl-4-imidazolyl)méthyl]-9-acridanone-(2-thiazolidinylidène)-hydrazone, la 10-[2-[4-[2-(diméthylamino)acétyl]-1-pipérazinyl]-éthyl]-9-acridanone-(2-thiazolidinylidène)-hydrazone, la 1-chloro-10-[2-(diéthylamino)éthyl]-9-acridanone-(2-thiazolidinylidène)-hydrazone, la 10-(2-aminoéthyl)-9-acridanone-(2-thiazolidinylidène)-hydrazone, la 10-[2-[(méthylsulfonyl)amino]éthyl]-9-acridanone-(2-thiazolidinylidène)-hydrazone, la 10-[4-(diéthylamino)butyl]-9-acridanone-(2-thiazolidinylidène)-hydrazone, la 10-[5-(diéthylamino)pentyl]-9-acridanone-(2-thiazolidinylidène)-hydrazone, la 10-[2-(diéthylamino)éthyl]-9-acridanone-méthyl-(2-thiazoline-2-yl)-hydrazone et la 10-[2-(diéthylamino)éthyl]-9-acridanone-(3-méthyl-2-thiazolidinylidène)-hydrazone.

23. La 10-[2-(diéthylamino)éthyl]-9-acridanone-méthyl-(2-thiazolyl)-hydrazone, la 10-[2-(4-morpholinyl)éthyl]-9-acridanone-(2-thiazolyl)-hydrazone, la 10-[2-(4-éthoxycarbonyl-1-pipérazinyl)éthyl]-9-acridanone-(2-thiazolyl)-hydrazone, la 10-[2-(4-pivaloyl-1-pipérazinyl)éthyl]-9-acridanone-(2-thiazolidinylidène)-hydrazone,

la
10-[2-[4-(méthylsulfonyl)-1-pipérazinyl]-éthyl]-9-acridanone-(2-thiazolyl)-hydrazone,
la
10-[2-[4-[2-(diéthylamino)éthyl]-1-pipérazinyl]éthyl]-9-acridanone-(2-thiazolyl)-hydrazone et
la
10-[2-(4-propyl-1-pipérazinyl)éthyl]-9-acridanone-(2-thiazolyl)-hydrazone.

24. Composés selon l'une des revendications 1 à 23, en tant que substances actives pharmaceutiques.

25. Composés selon l'une des revendications 1 à 23, en tant que substances schistosomicides.

26. Procédé de préparation des composés selon l'une des revendications 1 à 23, caractérisé en ce que:

a) on cyclise un composé de formule générale

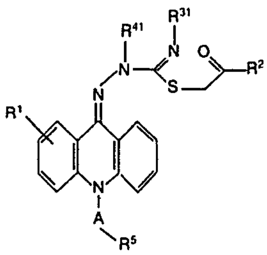
II,

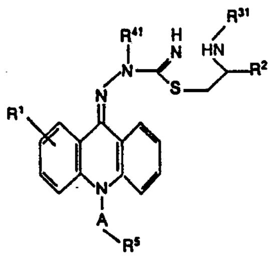
III,

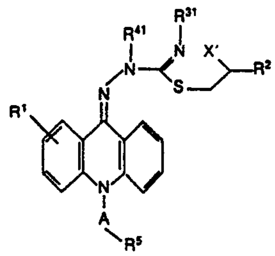
IV,

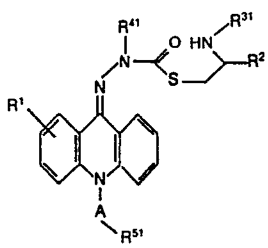
V,

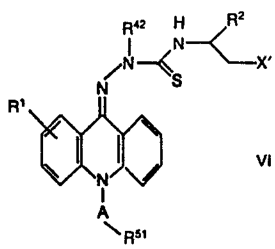
VI ou

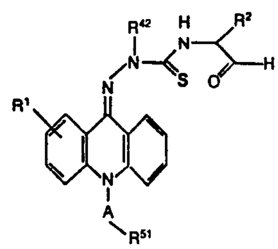
VII

dans laquelle l'un des symboles $R^{31}$ et $R^{41}$ représente l'hydrogène ou un groupe alkyle inférieur et l'autre représente l'hydrogène, $R^{42}$ représente l'hydrogène ou un groupe alkyle inférieur, $R^{51}$ a les significations indiquées pour $R^5$ dans la revendication 1 mais ne contient pas de groupe amino basique primaire ou secondaire et X' représente un groupe éliminable, et $R^1$, $R^2$ et $R^5$ ont les significations indiquées dans la revendication 1, ou bien

b) on fait réagir un composé de formule générale

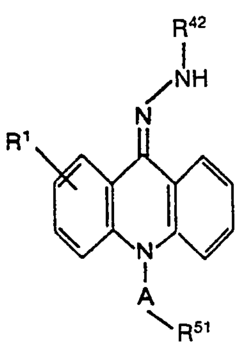
VIII

dans laquelle A et $R^1$ ont les significations indiquées dans la revendication 1 et $R^{42}$ et $R^{51}$ ont les significations indiquées ci-dessus,
avec un composé de formule générale

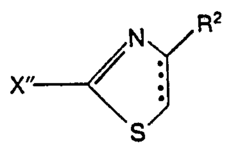
IX

dans laquelle X'' représente un groupe éliminable et le trait en pointillé et $R^2$ ont les significations indiquées dans la revendication 1, ou bien

c) on fait réagir un composé de formule générale

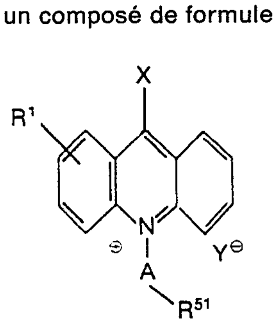
X

dans laquelle $Y^{\ominus}$ représente un anion et X un groupe éliminable, A et $R^1$ ayant les significations indiquées dans la revendication 1 et $R^{51}$ les significations indiquées ci-dessus,
avec un composé de formule générale

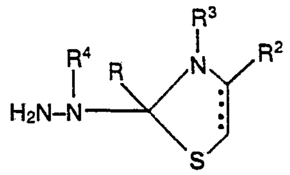

XI

dans laquelle le trait en pointillé, R, $R^2$, $R^3$ et $R^4$ ont les significations indiquées dans la revendication 1, ou bien

d) dans un composé de formule générale

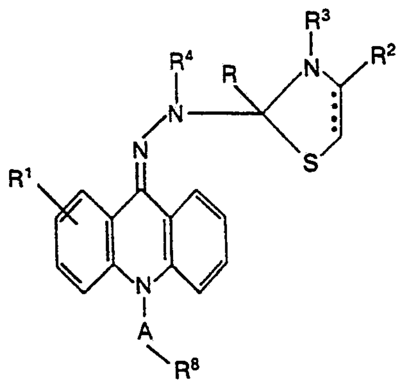

XII

dans laquelle $R^8$ a les significations indiquées pour $R^5$ dans la revendication 1 mais contient un groupe amino basique primaire ou secondaire bloqué par un groupe protecteur à l'azote, et le trait en pointillé, R, $R^1$, $R^2$, $R^3$ et $R^4$ ont les significations indiquées dans la revendication 1,
on élimine le groupe protecteur à l'azote, et

e) si on le désire, on convertit un composé obtenu répondant à la formule I en un sel acceptable pour l'usage pharmaceutique formé par addition avec un acide.

27. Médicament contenant un composé selon l'une des revendications 1 à 23.
28. Agent schistosomicide contenant un composé selon l'une des revendications 1 à 23.
29. Utilisation de composés selon l'une des revendications 1 à 23 pour la préparation de produits selon la revendication 27 ou 28.

**Revendications pour l'Etat Contractant AT**

1. Procédé de préparation des dérivés de l'acridanone répondant à la formule générale

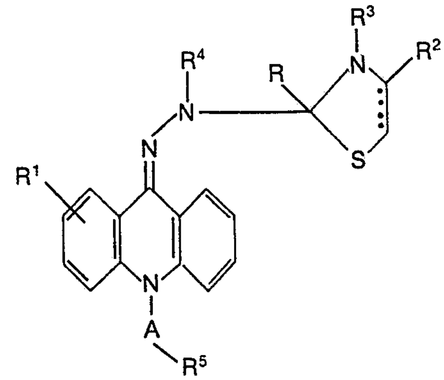

I

dans laquelle le trait en pointillé représente une liaison facultative, $R^1$ représente l'hydrogène, un halogène ou un groupe nitro, $R^2$ représente l'hydrogène ou un groupe alkyle inférieur, l'un des symboles $R^3$ et $R^4$ représente l'hydrogène ou un groupe alkyle inférieur, et l'autre forme avec R une liaison supplémentaire, A représente un groupe alkylène inférieur, $R^5$ représente un hétérocycle aromatique azoté à 5 chaînons éventuellement substitué par un groupe alkyle inférieur, un groupe amino ou un groupe

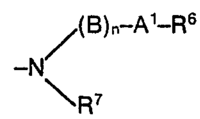

ou

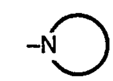

le symbole 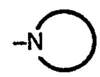

représente un hétérocycle saturé à 5 ou 6 chaînons, éventuellement substitué par un groupe alkyle inférieur et qui peut encore contenir en tant que chaînon cyclique un atome d'oxygène ou de soufre ou le groupe $>NH$ ou $>N(B)_n-A^1-R^6$, B représente un groupe –CO–, –COO– ou $-SO_2-$, n est égal à 0 ou 1, $A^1$ représente un groupe alkylène inférieur, $R^6$ représente l'hydrogène, un groupe amino, alkylamino inférieur ou di-(alkyle inférieur)-amino et $R^7$ représente l'hydrogène ou un groupe alkyle inférieur, les groupes qualifiés d'«inférieurs» contenant de 1 à 7 atomes de carbone, et de leurs sels acceptables pour l'usage pharmaceutique formés par addition avec des acides, caractérisé en ce que

a) on cyclise un composé de formule générale

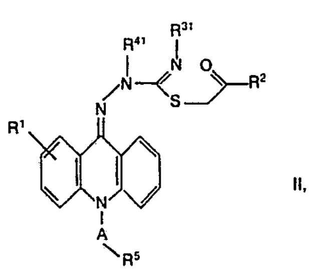

II,

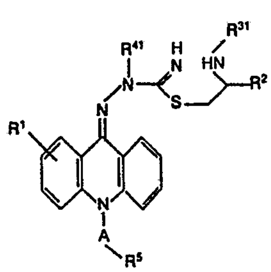

III,

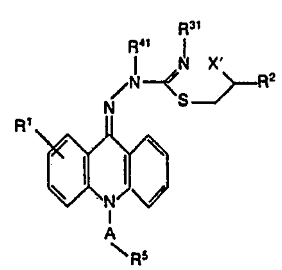

IV.

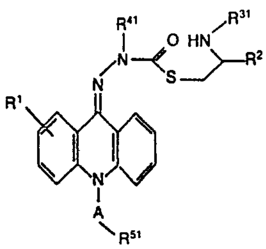

V,

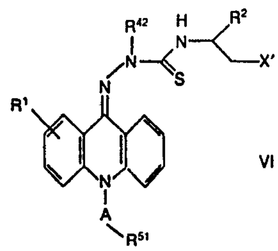

VI ou

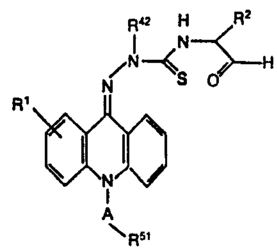

VII

dans laquelle l'un des symboles $R^{31}$ et $R^{41}$ représente l'hydrogène ou un groupe alkyle inférieur et l'autre représente l'hydrogène, $R^{42}$ représente l'hydrogène ou un groupe alkyle inférieur, $R^{51}$ a les significations indiquées ci-dessus pour $R^5$ mais ne contient pas de groupe amino basique primaire ou secondaire et X' représente un groupe éliminable, et $R^1$, $R^2$ et $R^5$ ayant les significations indiquées ci-dessus ou bien

b) on fait réagir un composé de formule générale

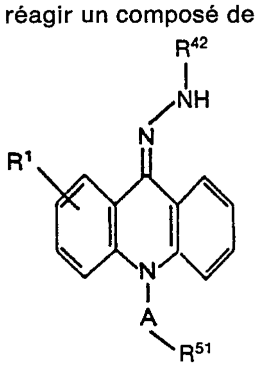

VIII

dans laquelle A, $R^1$, $R^{42}$ et $R^{51}$ ont les significations indiquées ci-dessus,
avec un composé de formule générale

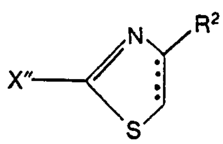

IX

dans laquelle X'' représente un groupe éliminable le trait en pointillé et $R^2$ ont les significations indiquées ci-dessus, ou bien

c) ont fait réagir un composé de formule générale

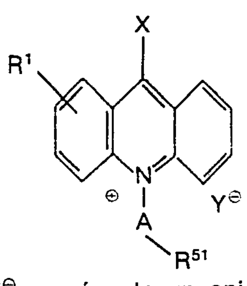

X

dans laquelle $Y^{\ominus}$ représente un anion et X un groupe éliminable, A, $R^1$ et $R^{51}$ ayant les significations indiquées ci-dessus,
avec un composé de formule générale

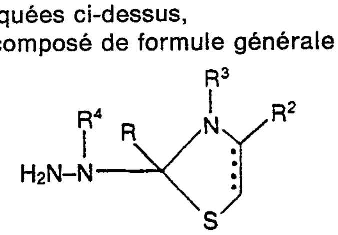

XI

dans laquelle le trait en pointillé, R, $R^2$, $R^3$ et $R^4$ ont les significations indiquées ci-dessus, ou bien

d) dans un composé de formule générale

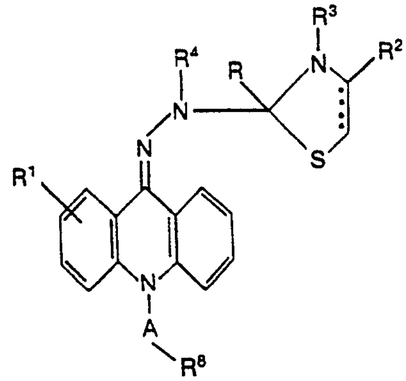

XII

dans laquelle $R^8$ a les significations indiquées ci-dessus pour $R^5$ mais contient un groupe amino basique primaire ou secondaire bloqué par un groupe protecteur à l'azote, et le trait en pointillé, R, $R^1$, $R^2$, $R^3$ et $R^4$ ont les significations indiquées ci-dessus,
on élimine le groupe protecteur à l'azote, et

e) si on le désire, on convertit un composé obtenu répondant à la formule I en un sel acceptable pour l'usage pharmaceutique formé par addition avec un acide.

2. Procédé selon la revendication 1, caractérisé en ce que l'on prépare un composé de formule I dans laquelle un substituant éventuel $R^1$ se trouve en position 1.

3. Procédé selon la revendication 1 ou 2, caractérisé en ce que l'on prépare un composé de formule I dans laquelle $R^1$ représente l'hydrogène.

4. Procédé selon l'une des revendications 1 à 3, caractérisé en ce que l'on prépare un composé de formule I dans laquelle $R^2$ représente l'hydrogène.

5. Procédé selon l'une des revendications 1 à 4, caractérisé en ce que l'on prépare un composé de formule I dans laquelle l'un des symboles $R^3$ et $R^4$ représente l'hydrogène et l'autre forme avec R une liaison supplémentaire.

6. Procédé selon l'une des revendications 1 à 5, caractérisé en ce que l'on prépare un composé de formule I dans laquelle A représente un groupe diméthylène ou triméthylène.

7. Procédé selon l'une des revendications 1 à 6, caractérisé en ce que l'on prépare un composé de formule I dans laquelle $R^5$ représente un groupe amino, 1-pyrrolidinyle, 1-pipéridinyle, 1-morpholinyle, 1-pipérazinyle ou le groupe $-NR^7-A^1-R^{61}$ ou

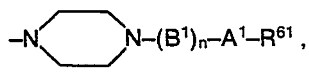

$A^1$ représente un groupe alkylène inférieur, $B^1$ représente le groupe –CO–, n est égal à 0 ou 1, $R^{61}$ représente l'hydrogène et $R^7$ l'hydrogène ou un groupe alkyle inférieur.

8. Procédé selon la revendication 7, caractérisé en ce que l'on prépare un composé de formule I dans laquelle $R^5$ représente un groupe amino, diméthylamino, diéthylamino, 1-pyrrolidinyle, 1-pipéridinyle, 4-méthyl-1-pipérazinyle ou 4-acétyl-1-pipérazinyle.

9. Procédé selon la revendication 1, caractérisé en ce que l'on prépare la
10-[2-(4-méthyl-1-pipérazinyl)éthyl]-9-acridanone-(2-thiazolyl)-hydrazone.

10. Procédé selon la revendication 1, caractérisé en ce que l'on prépare la
10-[2-(4-méthyl-1-pipérazinyl)éthyl]-9-acridanone-(2-thiazolidinylidène)-hydrazone.

11. Procédé selon la revendication 1, caractérisé en ce que l'on prépare la
10-[2-(4-acéthyl-1-pipérazinyl)éthyl]-9-acridanone-(2-thiazolidinylidène)-hydrazone.

12. Procédé selon la revendication 1, caractérisé en ce que l'on prépare la
10-[2-(diméthylamion)éthyl]-9-acridanone-(2-thiazolidinylidène)-hydrazone.

13. Procédé selon la revendication 1, caractérisé en ce que l'on prépare la
10-[2-(diéthylamino)éthyl]-9-acridanone-(2-thiazolyl)-hydrazone.

14. Procédé selon la revendication 1, caractérisé en ce que l'on prépare la
10-[2-(1-pipéridinyl)éthyl]-9-acridanone-(2-thiazolyl)-hydrazone.

15. Procédé selon la revendication 1, caractérisé en ce que l'on prépare la
10-(2-aminoéthyl)-9-acridanone-(2-thiazolyl)-hydrazone.

16. Procédé selon la revendication 1, caractérisé en ce que l'on prépare la
10-[2-(diméthylamino)éthyl]-9-acridanone-(2-thiazolyl)-hydrazone.

17. Procédé selon la revendication 1, caractérisé en ce que l'on prépare la
10-[2-(4-acétyl-1-pipérazinyl)éthyl]-9-acridanone-(2-thiazolyl)-hydrazone.

18. Procédé selon la revendication 1, caractérisé en ce que l'on prépare la
10-[3-(diméthylamino)propyl]-9-acridanone-(2-thiazolyl)-hydrazone.

19. Procédé selon la revendication 1, caractérisé en ce que l'on prépare la
10-[2-(diéthylamino)éthyl]-9-acridanone-(2-thiazolidinylidène)-hydrazone.

20. Procédé selon la revendication 1, caractérisé en ce que l'on prépare la
10-[2-(1-pyrrolidinyl)éthyl]-9-acridanone-(2-thiazolidinylidène)-hydrazone.

21. La 10-[3-(diméthylamino)propyl]-9-acridanone-(2-thiazolidinylidène)-hydrazone.

22. Procédé selon la revendication 1, caractérisé en ce que l'on prépare:
10-[2-(4-propyl-1-pipérazinyl)éthyl]-9-acridanone-(2-thiazolidinylidène)-hydrazone,
10-[2-(diméthylamino)propyl]-9-acridanone-(2-thiazolidinylidène)-hydrazone,
10-[2-(1-pipéridinyl)éthyl]-9-acridanone-(2-thiazolidinylidène)-hydrazone,
10-[2-(2,6-diméthyl-1-pipéridinyl)éthyl]-9-acridanone-(2-thiazolidinylidène)-hydrazone,
10-[2-(4-morpholinyl)éthyl]-9-acridanone-(2-thiazolidinylidène)-hydrazone,
10-[2-(4-éthoxycarbonyl-1-pipérazinyl)-éthyl]-9-acridanone-(2-thiazolidinylidène)-hydrazone,
10-[2-(4-pivaloyl-1-pipérazinyl)éthyl]-9-acridanone-(2-thiazolidinylidène)-hydrazone,
10-[2-[4-(méthylsulfonyl-1-pipérazinyl)-éthyl]-9-acridanone-(2-thiazolidinylidène)-hydrazone,
10-[(1-méthyl-4-imidazolyl)méthyl]-9-acridanone-(2-thiazolidinylidène)-hydrazone,
10-[2-[4-[2-(diméthylamino)acétyl]-1-pipérazinyl]-éthyl]-9-acridanone-(2-thiazolidinylidène)-hydrazone,
1-chloro-10-[2-(diéthylamino)éthyl]-9-acridanone-(2-thiazolidinylidène)-hydrazone,
10-(2-aminoéthyl)-9-acridanone-(2-thiazolidinylidène)-hydrazone,
10-[2-[(méthylsulfonyl)amino]éthyl]-9-acridanone-(2-thiazolidinylidène)-hydrazone,
10-[4-(diéthylamino)butyl]-9-acridanone-(2-thiazolidinylidène)-hydrazone,
10-[5-(diéthylamino)pentyl]-9-acridanone-(2-thiazolidinylidène)-hydrazone,
10-[2-(diéthylamino)éthyl]-9-acridanone-méthyl(2-thiazoline-2-yl)-hydrazone et
10-[2-(diéthylamino)éthyl]-9-acridanone-(3-méthyl-2-thiazolidinylidène)-hydrazone.

23. Procédé selon la revendication 1, caractérisé en ce que l'on prépare:
10-[2-(diéthylamino)éthyl]-9-acridanone-méthyl-(2-thiazolyl)-hydrazone,
10-[2-(4-morpholinyl)éthyl]-9-acridanone-(2-thiazolyl)-hydrazone,
10-[2-(4-éthoxycarbonyl-1-pipérazinyl]-éthyl]-9-acridanone-(2-thiazolyl)-hydrazone,
10-[2-(4-pivaloyl-1-pipérazinyl)éthyl]-9-acridanone-(2-thiazolidinylidène)-hydrazone,
10-[2-[4-(méthylsulfonyl)-1-pipérazinyl]-éthyl]-9-acridanone-(2-thiazolyl)-hydrazone,
10-[2-[4-[2-(diéthylamino)éthyl]-1-pipérazinyl]éthyl]-9-acridanone-(2-thiazolyl)-hydrazone et
10-[2-(4-propyl-1-pipérazinyl)éthyl]-9-acridanone-(2-thiazolyl)-hydrazone.

24. Utilisation des composés de formule I, définie dans la revendication 1, pour la préparation de médicaments.

25. Utilisation des composés de formule I définie dans la revendication 1, pour la préparation d'agents schistosomicides.